# FASCICULE DE BREVET EUROPEEN

(11) **EP 2 593 485 B1**
(45) Date de publication et mention de la délivrance du brevet: **23.03.2016**
(21) Numéro de dépôt: 11730680.3
(22) Date de dépôt: 12.07.2011
(51) Int. Cl.: C08C 19/28, C08C 19/22, C07D 233/34, C07D 233/36

(54) **POLYMERE GREFFE PAR DES MOLECULES ASSOCIATIVES AZOTEES**
MIT ASSOZIERTEN STICKSTOFFMOLEKÜLEN GEKRAFTETE POLYMERE
POLYMERS CRAFTET WITH AZOTIC MOLECULES

(30) Priorité: 13.07.2010 FR 1002954
(43) Date de publication de la demande: 22.05.2013
(73) Titulaire: Compagnie Générale des Etablissements Michelin, 63000 Clermont-Ferrand (FR); MICHELIN Recherche et Technique S.A., 1763 Granges-Paccot (CH)
(72) Inventeur: ARAUJO, José, F-63040 CLERMONT-FERRAND Cedex 9 (FR); FAVROT, Jean-Michel, F-63040 CLERMONT-FERRAND Cedex 9 (FR); SALIT, Anne-Frédérique, F-63040 CLERMONT-FERRAND Cedex 9 (FR); SEEBOTH, Nicolas, F-63040 CLERMONT-FERRAND cedex 9 (FR)
(74) Mandataire: Le Cam, Véronique Marie Christine
(86) Numéro de dépôt international: PCT/EP2011/061799
(87) Numéro de publication internationale: WO 2012/007441

(56) Documents cités:
- JP-A- 2008 208 163
- US-A1- 2004 106 744
- US-A1- 2006 084 730
- US-A1- 2006 199 917
- MAURIZIO GALIMBERTI ET AL: "Elastomeric Compounds with Silica. Lower Hysteresis in the Presence of Functionalised Isoprene Oligomers", MACROMOLECULAR SYMPOSIA, WEINHEIM, vol. 234, 13 mars 2006 (2006-03-13), pages 203-210, XP002612228,

## Description

La présente invention concerne un polymère modifié par greffage de molécules associatives azotées comprenant au moins un motif les rendant susceptibles de s'associer entre elles ou avec une charge par des liaisons non covalentes

Depuis que les économies de carburant et la nécessité de préserver l'environnement sont devenues une priorité, il est souhaitable de produire des mélanges possédant de bonnes propriétés mécaniques et une hystérèse aussi faible que possible afin de pouvoir les mettre en oeuvre sous forme de compositions de caoutchouc utilisables pour la fabrication de divers produits semi-finis entrant dans la composition d'enveloppes de pneumatique, tels que par exemple des sous-couches, des flancs, des bandes de roulement, et afin d'obtenir des pneumatiques possédant une résistance au roulement réduite.

La réduction de l'hystérèse des mélanges est un objectif permanent qui doit toutefois se faire en conservant l'aptitude à la mise en oeuvre des mélanges.

Pour atteindre l'objectif de baisse d'hystérèse, de nombreuses solutions ont déjà été expérimentées. En particulier, on peut citer la modification de la structure des polymères et des copolymères diéniques en fin de polymérisation au moyen d'agents de fonctionnalisation, de couplage ou d'étoilage ou post-polymérisation dans le but d'obtenir une bonne interaction entre le polymère ainsi modifié et la charge, qu'il s'agisse du noir de carbone ou d'une charge inorganique renforçante.

Par exemple, les demandes de brevets EP-A-0 590 491 et EP-A-0 593 049 décrivent des polymères portant des fonctions amines permettant une meilleure interaction entre le polymère et le noir de carbone.

On peut également citer les brevets US 5 015 692 ou encore le brevet EP 0 341 496 qui proposent un procédé de fonctionnalisation d'élastomères par des composés tels que des composés phosphorés, nitrés, des aminosilanes, des acrylamides, des aminovinylsilanes, le cas échéant combiné à un couplage ou un étoilage avec un composé à base de silicium ou d'étain, en vue d'améliorer l'élasticité au choc de compositions de caoutchouc les contenant. Ces brevets décrivent notamment des compositions de caoutchouc renforcées au noir de carbone contenant un polybutadiène fonctionnalisé en bout de chaîne par un alcoxysilane portant un groupement amine.

Dans le cadre de la fonctionnalisation post-polymérisation, les documents US 7,186,845 B2 et JP2008208163 décrivent également la modification de polymères comprenant des motifs diéniques par des composés dipolaires azotés comprenant en outre un hétérocycle, ledit hétérocycle comprenant lui-même un atome d'azote, et un atome d'oxygène et/ou de soufre. Plus particulièrement les composés décrits sont des nitrones porteuses de fonction oxazolines, thiazolines comme par exemple la (-(2-oxazolyl)- Phenyl-N-methylnitrone)

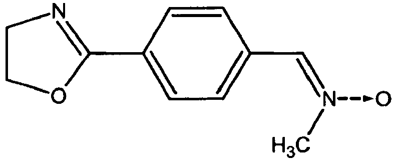

Lorsque des polymères diéniques sont amenés à réagir avec de tels composés, les polymères en résultant porteront les cycles oxazoline ou thiazoline.

Ces cycles présents sur le polymère sont susceptibles de réagir, à leur tour, avec des fonctions de surface des charges (comme le noir de carbone ou la silice) avec lesquels les polymères sont mélangés. Cette réaction conduit à l'établissement de liaisons covalentes entre le polymère modifié par l'agent de couplage et la charge du fait de l'ouverture du cycle oxazoline ou thiazoline. En effet, comme cela est décrit dans le document US7186845B2, les cycles oxazolines et ou thioazolines sont susceptibles de s'ouvrir en présence d'un nucléophile pouvant par exemple être présent à la surface de la charge.

Ces polymères confèrent notamment aux compositions de caoutchouc qui les contiennent une hystérèse réduite.

Par ailleurs, le document publié sous le numéro WO2010/031956 décrit des élastomères fonctionnalisés par des hétérocycles azotés. L'obtention de tels polymères modifiés est réalisée grâce à l'addition d'agents de modification possédant un groupement réactif d'une part et un hétérocycle azoté d'autre part. Le greffage du groupement réactif de l'agent de modification se fait sur des fonctions déjà présentes sur le polymère telles que des époxys, des acides, des anhydride etc...L'obtention d'élastomères possédant des hétérocycles azotés requiert donc l'utilisation d'élastomères préfonctionnalisés.

Parmi les groupements associatifs envisagés dans ce document sont cités les groupes imidazolidinyle, triazolyle, triazinyle, bis-uréyle, uréido-pyrimidyle.

De manière inattendue, la demanderesse a trouvé que la modification de polymère par des groupes, autres que les thiazolines et oxazolines cités dans les brevets ci-dessus, peu sensibles aux attaques nucléophiles permet d'obtenir une diminution de l'hystérèse par rapport à un polymère non modifié.

L'invention a pour objet un polymère modifié par greffage d'un composé comprenant au moins un groupement Q, et au moins un groupement A reliés entre eux par au moins et de préférence un groupement « espaceur » Sp dans lequel :
- Q comprend un dipôle contenant au moins et de préférence un atome d'azote,
- A comprend un groupe associatif comprenant au moins un atome d'azote, ≤ le groupe associatif étant choisi parmi un groupe imidazolidinyle, triazolyle, uréyle, bis-uréyle, uréido-pyrimidyle
- Sp est un atome ou un groupe d'atomes formant une liaison entre Q et A.

Un polymère modifié par greffage d'un composé tel que défini ci-dessus mélangé à des charges permet d'assurer une bonne interaction polymère - charge, bénéfique pour les propriétés finales du polymère,

L'invention a également pour objet un procédé permettant de préparer le polymère modifié défini ci-dessus par greffage de composés comprenant des groupes associatifs azotés avec un bon rendement de greffage.

Ainsi l'invention a pour objet un polymère modifié par greffage d'un composé comprenant au moins un groupement Q, et au moins un groupement A reliés entre eux par au moins et de préférence un groupement « espaceur » Sp tels que décrits plus haut.

Par polymère, on entend selon l'invention, tout polymère contenant au moins une insaturation ou double liaison susceptible de réagir sur le composé décrit ci-dessus.

De préférence, les polymères de la présente invention sont des élastomères diéniques.

Ces élastomères diéniques peuvent être classés de manière connue en deux catégories, ceux dits essentiellement insaturés et ceux dits essentiellement saturés. Ces deux catégories d'élastomères diéniques sont envisageables dans le cadre de l'invention.

Un élastomère diénique essentiellement saturé a un taux de motifs ou unités d'origine diénique faible ou très faible (diènes conjugués) qui est toujours inférieur à 15% (% en moles). C'est ainsi, par exemple, que les caoutchoucs butyle ou les copolymères de diènes et d'alpha-oléfines type EPDM entrent dans la définition d'élastomères diéniques essentiellement saturés.

A contrario, par élastomère diénique essentiellement insaturé, on entend un élastomère diénique issu au moins en partie de monomères diènes conjugués, ayant un taux de motifs ou unités d'origine diénique (diènes conjugués) qui est supérieur à 15% (% en moles). Dans la catégorie des élastomères diéniques "essentiellement insaturés", on entend en particulier par élastomère diénique "fortement insaturé" un élastomère diénique ayant un taux de motifs d'origine diénique (diènes conjugués) qui est supérieur à 50%.

On entend plus particulièrement par élastomère diénique susceptible d'être utilisé dans l'invention:
(a) - tout homopolymère obtenu par polymérisation d'un monomère diène conjugué ayant de 4 à 12 atomes de carbone;
(b) - tout copolymère obtenu par copolymérisation d'un ou plusieurs diènes conjugués entre eux ou avec un ou plusieurs composés vinyle aromatique ayant de 8 à 20 atomes de carbone;
(c) - un copolymère ternaire obtenu par copolymérisation d'éthylène, d'une α-oléfine ayant 3 à 6 atomes de carbone avec un monomère diène non conjugué ayant de 6 à 12 atomes de carbone, comme par exemple les élastomères obtenus à partir d'éthylène, de propylène avec un monomère diène non conjugué du type précité tel que notamment l'hexadiène-1,4, l'éthylidène norbornène, le dicyclopentadiène; de tels polymères sont décrits en particulier dans les documents WO 2004/035639A1 et US 2005/0239639A1 ;
(d) - un copolymère d'isobutène et d'isoprène (caoutchouc butyle), ainsi que les versions halogénées, en particulier chlorées ou bromées, de ce type de copolymère.

Bien qu'elle s'applique à tout type d'élastomère diénique, on préfère utiliser au moins un élastomère diénique du type fortement insaturé en particulier du type (a) ou (b) ci-dessus.

A titre de diènes conjugués conviennent notamment le butadiène-1,3, le 2-méthyl-1,3-butadiène, les 2,3-di(alkyle en C1-C5)-1,3-butadiènes tels que par exemple le 2,3-diméthyl-1,3-butadiène, le 2,3-diéthyl-1,3-butadiène, le 2-méthyl-3-éthyl-1,3-butadiène, le 2-méthyl-3-isopropyl-1,3-butadiène, un aryl-1,3-butadiène, le 1,3-pentadiène, le 2,4-hexadiène. A titre de composés vinylaromatique conviennent par exemple le styrène, l'ortho-, méta-, para-méthylstyrène, le mélange commercial "vinyle-toluène", le para-tertiobutylstyrène, les méthoxystyrènes, les chlorostyrènes, le vinylmésitylène, le divinylbenzène, le vinylnaphtalène.

Les copolymères peuvent contenir entre 99% et 20% en poids d'unités diéniques et entre 1% et 80% en poids d'unités vinylaromatique. Les élastomères peuvent avoir toute microstructure qui est fonction des conditions de polymérisation utilisées, notamment de la présence ou non d'un agent modifiant et/ou randomisant et des quantités d'agent modifiant randomisant employées. Les élastomères peuvent être par exemple à blocs, statistiques, séquencés, microséquencés, et être préparés en dispersion, en émulsion ou en solution ; ils peuvent être couplés et/ou étoilés ou encore fonctionnalisés avec un agent de couplage et/ou d'étoilage ou de fonctionnalisation.

Conviennent en particulier les élastomères diéniques choisis dans le groupe constitué par les polybutadiènes (BR), les polyisoprènes de synthèse (IR), le caoutchouc naturel (NR), les copolymères de butadiène, les copolymères d'isoprène et les mélanges de ces élastomères. De tels copolymères sont plus préférentiellement choisis dans le groupe constitué par les copolymères de butadiène-styrène (SBR), les copolymères d'isoprène-butadiène (BIR), les copolymères d'isoprène- styrène (SIR), les copolymères d'isoprène-butadiène-styrène (SBIR) et les mélanges de tels copolymères.

Selon l'invention, le polymère possédant au moins une insaturation ou double liaison est modifié par greffage d'un composé, appelé également agent de modification, comprenant au moins un groupement Q, et au moins un groupement A reliés entre eux par au moins et de préférence un groupement « espaceur » Sp dans lequel :
- Q comprend un dipôle contenant au moins et de préférence un atome d'azote,
- A comprend un groupe associatif comprenant au moins un atome d'azote,, ≤ le groupe associatif étant choisi parmi un groupe imidazolidinyle, triazolyle, uréyle, bis-uréyle, uréido-pyrimidyle
- Sp est un atome ou un groupe d'atomes formant une liaison entre Q et A.

On entend par dipôle une fonction capable de former une addition dipolaire 1,3 sur une liaison carbone-carbone insaturée.

Par « groupe associatif », on entend des groupes susceptibles de s'associer les uns aux autres par des liaisons hydrogène, ioniques et/ou hydrophobes. Il s'agit selon un mode préféré de l'invention de groupes susceptibles de s'associer par des liaisons hydrogène.

Lorsque les groupes associatifs sont susceptibles de s'associer par des liaisons hydrogène, chaque groupe associatif comporte au moins un « site » donneur et un site accepteur vis-à-vis de la liaison hydrogène de sorte que deux groupes associatifs identiques sont auto-complémentaires et peuvent s'associer entre eux en formant au moins deux liaisons hydrogène.

Les groupes associatifs selon l'invention sont également susceptibles de s'associer par des liaisons hydrogène, ioniques et/ou hydrophobes à des fonctions présentes sur des charges.

Les composés selon l'invention comportant un groupement Q, un groupement « espaceur » et un groupement associatif peuvent par exemple être représentés par la formule (Ia) suivante :

A - Sp - Q (Ia).

Les composés selon l'invention comportant un groupement Q, un groupement « espaceur » et deux groupements associatifs peuvent par exemple être représentés par la formule (Ib) suivante :

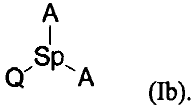

De manière similaire, les composés selon l'invention comportant deux groupements Q, un groupement « espaceur » et un groupement associatif peuvent par exemple être représentés par la formule (Ic) suivante :

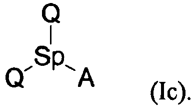

Selon le même principe, les composés selon l'invention comportant deux groupements Q, un groupement « espaceur » et deux groupements associatifs peuvent par exemple être représentés par la formule (Id) suivante :

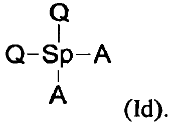

De préférence, le groupement A répond à l'une des formules (II) à (VI) suivantes : 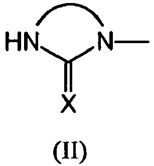 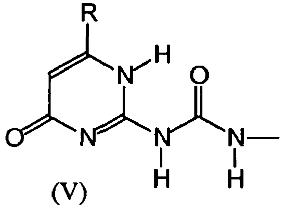 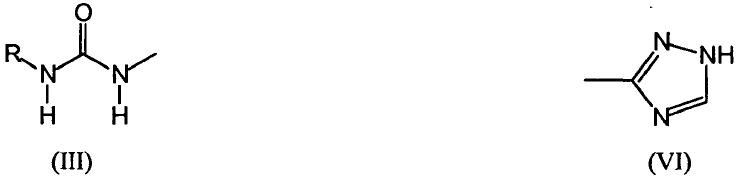 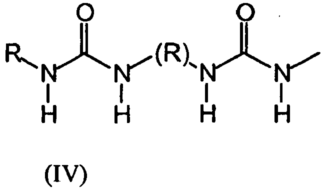 où :
- R désigne un groupement hydrocarboné pouvant éventuellement contenir des hétéroatomes,
- X désigne un atome d'oxygène, de soufre ou un groupement -NH, de préférence un atome d'oxygène.

De préférence, le groupement A comprend un hétérocycle di ou triazoté, à 5 ou 6 atomes, de préférence diazoté, et comprenant au moins une fonction carbonyle.

De manière encore plus préférée, le groupement A comprend un groupe imidazolidinyle de formule (II).

Le groupement Q est susceptible de se lier à une chaîne polymère comportant au moins une insaturation ou double liaison par liaison covalente (greffage). De préférence le groupement Q comprend une fonction oxyde de nitrile, nitrone, ou nitrile imine pouvant se lier à un polymère porteur d'au moins une insaturation, par une cycloaddition de type [3+2].

De préférence le groupement Q est un groupement de formule (VII), (VIII) ou (IX) suivante 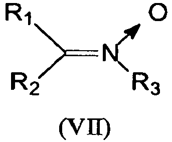 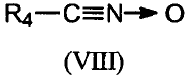 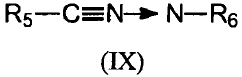 dans lesquelles R1 à R6 sont choisis indépendamment parmi un groupe espaceur Sp, un atome d'hydrogène, un groupe alkyle en C1-C20 linéaire ou ramifié, un groupe cycloalkyle en C3-C20 linéaire ou ramifié, un groupe aryle en C6-C20 linéaire ou ramifié, et un groupe de formule (X) 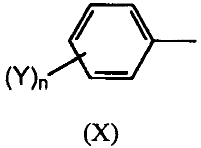 dans laquelle n représente 1, 2, 3, 4 ou 5 et chaque Y représente indépendamment un groupe espaceur Sp, un groupe alkyle ou un halogénure

Le groupement « espaceur » Sp permet de relier au moins un groupement Q et/ou au moins un groupement associatif, A, et ainsi peut être de tout type connu en soi. Le groupement « espaceur » ne doit cependant pas, ou peu, interférer avec les groupements Q et associatif du composé selon l'invention.

Ledit groupement « espaceur » est donc considéré comme un groupement inerte vis-à-vis du groupement Q. Le groupement « espaceur » est de préférence une chaîne hydrocarbonée, linéaire, ramifiée, cyclique, peut contenir un ou plusieurs radicaux aromatiques, et/ou un ou plusieurs hétéroatomes. Ladite chaîne peut éventuellement être substituée, pour autant que les substituants soient inertes vis-à-vis des groupements Q.

Selon un mode de réalisation préféré, le groupement « espaceur » est une chaîne alkyle linéaire ou ramifiée en C1-C24, de préférence C1-C10 et plus préférentiellement une chaîne alkyle linéaire en C1-C6, éventuellement comprenant un ou plusieurs hétéroatomes choisis parmi les atomes d'azote, de soufre, de silicium ou d'oxygène.

Selon un mode de réalisation de l'invention, le groupement Q est de préférence un groupement de formule (XIa) ou (XIb): 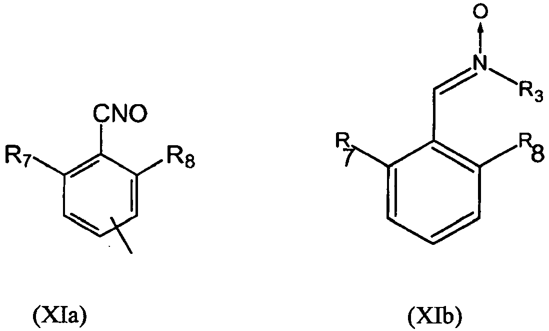 dans laquelle R7 et R8 représentent indépendamment un hydrogène ou un groupe alkyle en C1-C5, un alcoxyle ou un halogénure et de préférence R7 et R8 représentent indépendamment un groupe alkyle ou un halogénure, et plus préférentiellement R7 et R8 représentent indépendamment un groupe méthyle ou un atome de chlore, R3 est tel que défini plus haut et le groupement A est un groupement de formule (XII) :

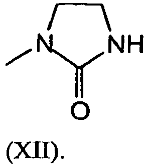

De préférence, le composé destiné à greffer le polymère conformément à l'invention est alors choisi parmi les composés de formule (XIII) à (XXI) suivantes:

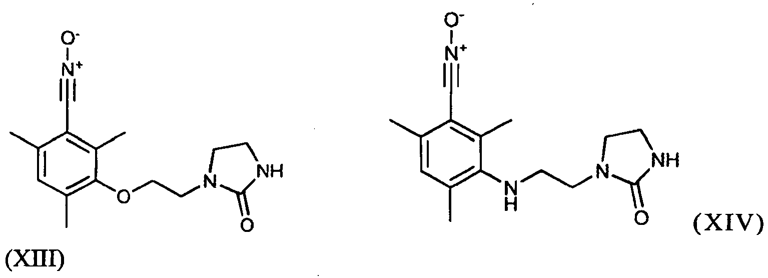

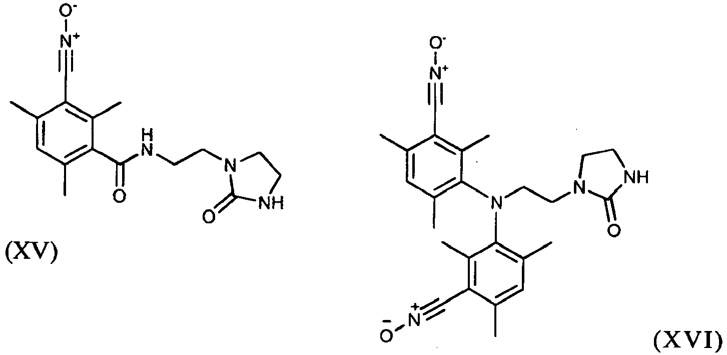

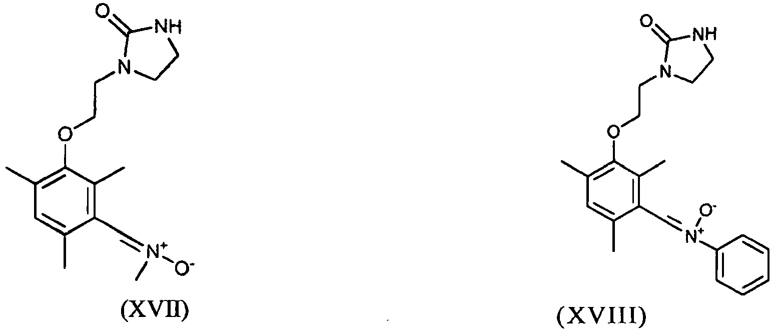

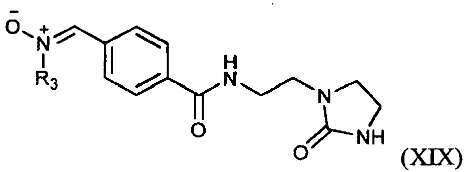

R3 est tel que défini plus haut

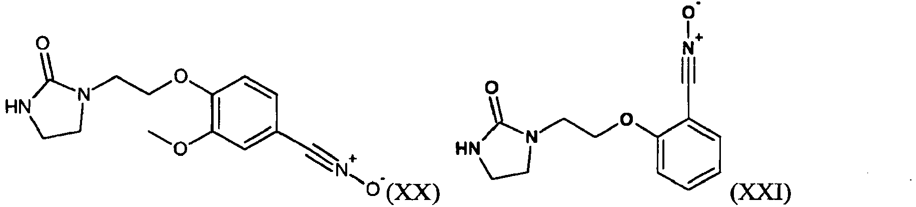

Selon un autre mode de réalisation de l'invention, le composé destiné à greffer le polymère conformément à l'invention est choisi parmi le composé de formule (XXII) à (XXIII) suivantes: 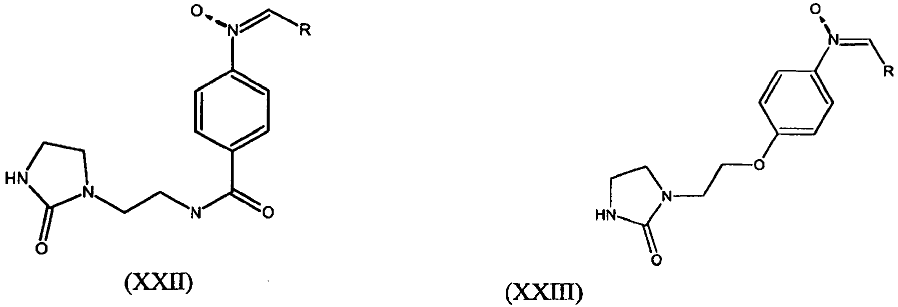 dans lesquelles

R est choisi parmi un groupe espaceur Sp, un atome d'hydrogène, un groupe alkyle en C1-C20 linéaire ou ramifié, un groupe cycloalkyle en C3-C20 linéaire ou ramifié, un groupe aryle en C6-C20 linéaire ou ramifié, et un groupe de formule (X) 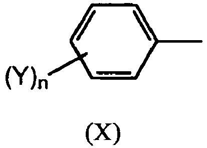 dans laquelle n représente 1, 2, 3, 4 ou 5 et chaque Y représente indépendamment un groupe espaceur Sp, un groupe alkyle ou un halogénure

Selon un mode de réalisation préféré, le taux d'agent de modification varie de 0,01 à 50% molaire, de préférence de 0,01 à 5% molaire.

L'invention a également pour objet le procédé de préparation de l'élastomère greffé décrit plus haut.

Le greffage du polymère se fait par réaction dudit polymère avec le ou les groupes réactifs portés par l'agent de modification. Lors de cette réaction, ce ou ces groupes réactifs forment des liaisons covalentes avec la chaîne du polymère. Le rendement de greffage est particulièrement élevé, préférentiellement supérieur à 60% et encore plus préférentiellement supérieur à 80%.

Le greffage de l'agent de modification est effectué par cycloaddition [3+2] du ou des groupes réactifs de l'agent de modification et une ou plusieurs doubles liaisons de la chaîne du polymère. Le mécanisme de la cycloaddition peut être illustré par les équations suivantes:
- Cycloaddition d'un oxyde de nitrile sur une insaturation ou double liaison du polymère (ici un polysisoprène) 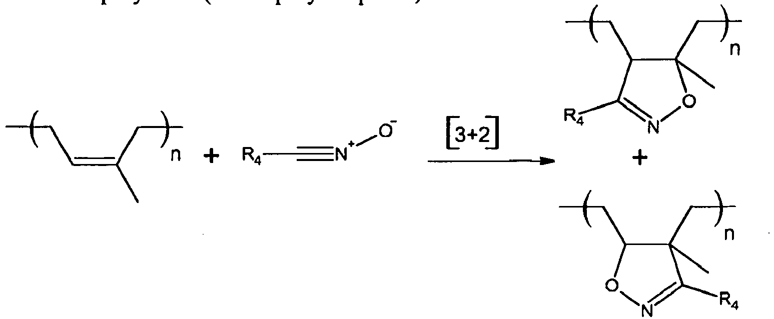
- Cycloaddition d'une nitrone sur une insaturation ou double liaison du polymère (ici un polyisoprène) 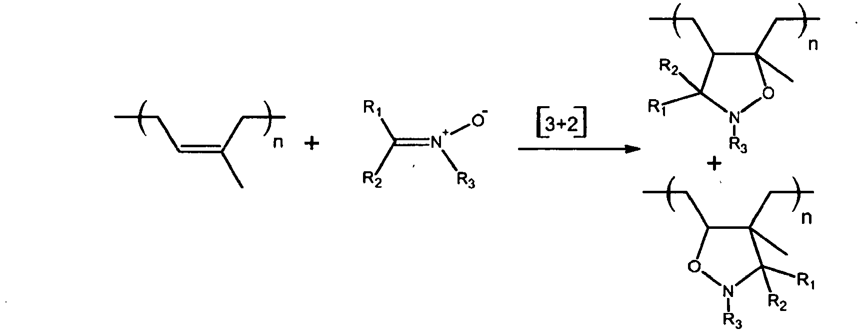
- Cycloaddition d'un nitrile imine sur une insaturation ou double liaison du polymère (ici un polyisoprène)

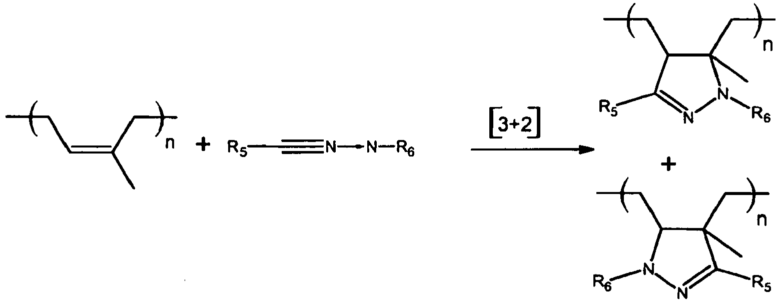

Les substituants R sont tels que décrits précédemment.

Le greffage de l'agent de modification peut être réalisé en masse, par exemple dans un mélangeur interne ou un mélangeur externe tel qu'un mélangeur à cylindres, ou en solution. Le procédé de greffage peut être effectué en solution en continu ou en discontinu. Le polymère ainsi modifié peut être séparé de sa solution par tout type de moyen connu par l'homme de l'art et en particulier par une opération de stripping à la vapeur d'eau.

L'invention ainsi que ses avantages seront aisément compris à la lumière des exemples de réalisation qui suivent.

### EXEMPLES DE REALISATION

L'analyse structurale ainsi que la détermination des puretés molaires des molécules de synthèses sont réalisées par une analyse RMN. Les spectres sont acquis sur un spectromètre Avance 500 MHz BRUKER équipé d'une sonde " large bande " BBIz-grad 5 mm. L'expérience RMN 1H quantitative, utilise une séquence simple impulsion 30° et un délai de répétition de 3 secondes entre chacune des 64 acquisitions. Les échantillons sont solubilisés dans le Diméthylsulfoxide deutéré (DMSO). Ce solvant est également utilisé pour le signal de lock. La calibration est réalisée sur le signal des protons du DMSO deutéré à 2.44ppm par rapport à une référence TMS à 0ppm. Le spectre RMN 1H couplé aux expériences 2D HSQC 1H/13C et HMBC 1H/13C permettent la détermination structurale des molécules (cf tableaux d'attributions). Les quantifications molaires sont réalisées à partir du spectre RMN 1D 1H quantitatif.

La mesure Infrarouge permet de valider la présence du groupement oxyde de nitrile porté par un aromatique. Les spectres sont acquis sur un spectromètre à transformée de Fourier VERTEX 70 équipé d'un détecteur DTGS. Les spectres sont acquis en 32 scans entre 4000cm-1 et 400cm-1 avec une résolution de 2cm-1. Les échantillons sont préparés sous forme de pastilles KBr. La fonction oxyde de nitrile portée par l'aromatique est caractérisée par une bande à 2295cm-1.

L'analyse par spectrométrie de masse est réalisée en injection directe par un mode d'ionisation en electrospray (ID/ESI). Les analyses ont été réalisées sur un spectromètre HCT Bruker (débit 600µL/min, Pression du gaz nébuliseur 10 psi, débit du gaz nébuliseur 4L/min).

La détermination du taux molaire de composé oxyde de nitrile greffé est réalisée par une analyse RMN. Les spectres sont acquis sur un spectromètre 500 MHz BRUKER équipé d'une sonde " large bande " BBIz-grad 5 mm. L'expérience RMN ¹H quantitative, utilise une séquence simple impulsion 30° et un délai de répétition de 3 secondes entre chaque acquisition. Les échantillons sont solubilisés dans le sulfure de carbone (CS₂). 100 µL de cyclohexane deutéré (C₆D₁₂) sont ajoutés pour le signal de lock.

Le spectre RMN ¹H permet de quantifier les motifs oxyde de nitrile greffés par intégration des signaux caractéristiques des protons CH₂N et CH₂O qui apparaissent à un déplacement chimique compris entre δ=3.1-3,8ppm

Le spectre RMN 2D HSQC ¹H-¹³C permet de vérifier la nature du motif greffé grâce aux déplacements chimiques des atomes de carbone et de proton.

### Exemple 1 : Modification d'un SBR par greffage de 2,4,6-trimethyl-3-(2-(2-oxoimidazolidin-1-yl)ethoxy) nitriloxide

### 1.1 - Préparation de l'agent de modification:

### a) Préparation du 1-(2-(3'-nitrileoxymesityl-1'-oxy)ethyl)imidazolidin-2-one

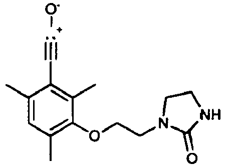

Ce composé peut être préparé à partir du mésitol de l'hydroxyethylimidazolidone selon le schéma synthétique suivant.

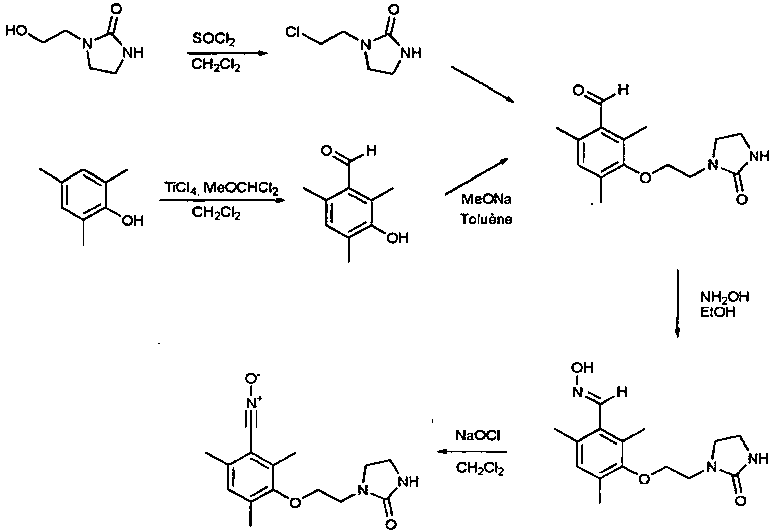

### b) Préparation du 3-hydroxy-2,4,6-trimethylbenzaldehyde

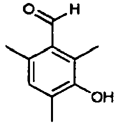

Ce composé peut être obtenu selon une procédure décrite dans l'article suivant : Yakubov, A. P.; Tsyganov, D. V.; Belen'kii, L. I.; Krayushkin, M. M.; Bulletin of the Academy of Sciences of the USSR, Division of Chemical Science (English Translation); vol. 40; nb. 7.2; (1991); p. 1427 - 1432*;* Izvestiya Akademii Nauk SSSR, Seriya Khimicheskaya; nb. 7; (1991); p. 1609-1615*.*

### c) Préparation de la 1-(2-chloroethyl)imidazolidin-2-one :

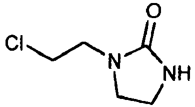

Ce produit est décrit dans l'article Nagarajan K., Arya V. P., Shah R. K.; Indian Journal of Chemistry, Section B: Organic Chemistry Including Medicinal Chemistry; 21; 10; 1982; 928-940*.*

A une solution de 1-(2-hydroxyethyl)imidazolidin-2-one (50,0 g, 0,39 mol) dans le dichlorométhane (250 mL) est ajouté goutte à goutte, à température ambiante, le chlorure de thionyle (34 mL, 0,47 mol) pendant 35 minutes. A la fin de l'addition la température du milieu réactionnel est de 35°C. Le milieu réactionnel est maintenu à une température de 35-40°C pendant 2.5 heures. Après évaporation sous pression réduite (T_{bain} 35 °C, 15-17 mbar) le produit brut est obtenu (67 g). Ce brut est cristallisé dans un mélange d'acétone et d'éther de pétrole (35 g pour 950 mL d'acétone et 820 mL d'éther de pétrole à -24 °C pendant 10 à 15 heures). Les cristaux sont filtrés, lavés par de l'éther de pétrole (2 fois par 40 mL) puis séchés pendant 10 à 15 heures sous pression atmosphérique à température ambiante.
Un solide blanc (33,3 g, rendement 66 %) de point de fusion 93 °C est obtenu.
La pureté molaire est supérieure à 97 % (RMN ¹H).

Une caractérisation RMN ¹H et ¹³C est présentée dans le tableau 1 suivant.

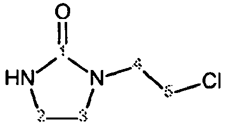

**Tableau 1**

| Atome | δ ¹H (ppm) + mult. | δ ¹³C (ppm) |
|---|---|---|
| 1 | - | 162.1 |
| 2 | 3.17 (t) | 37.5 |
| 3 | 3.33 (t) | 44.7 |
| 4 | 3.29 (t) | 45.0 |
| 5 | 3.62 (t) | 42.4 |

Solvant utilisé : DMSO - Calibration sur le signal du DMSO à 2.44 ppm en ¹H, 39.5 ppm en ¹³C.

### d) Préparation de 2,4,6-trimethyl-3-(2-(2-oxoimidazolidin-1-yl)ethoxy)benzaldehyde :

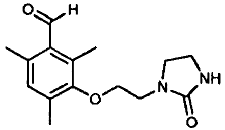

A une solution de sodium (1.63 g, 0.071 mol.) dans le méthanol (60 mL) est ajouté goutte à goutte le 3-hydroxy-2,4,6-trimethylbenzaldehyde (11.90 g, 0.073 mol.) dans le toluène anhydre (300 mL). Le mélange est porté au reflux puis le méthanol est distillé (volume de mélange azéotropique recueilli 80-90 mL). Après retour à 80-90 °C, la (2-chloroethyl)imidazolidin-2-one (10.45 g, 0,070 mol) est ajoutée en une fois au milieu réactionnel. Après 7 heures de reflux, les solvants sont évaporés sous pression réduite (T_{bain} 50 °C, 25 mbar). Au mélange obtenu est ajoutée du dichloromethane (150 mL) et de l'eau (30 mL). La phase organique est lavée ensuite 2 fois à l'eau (20 mL). Après séchage sur Na₂SO₄, le dichlorométhane est évaporé sous pression réduite (T_{bain} 35 °C, 33 mbar). Au mélange obtenu (24 g) est ajoutée de l'éther de pétrole (3 fois par 50 mL) et de l'eau (50 mL) et le précipite obtenu est filtré et lavé sur le filtre par de l'eau (15 mL) et de l'éther de pétrole (2 fois par 15 mL).
Le produit obtenu est repurifié par un lavage du produit en solution dans le dichlorométhane (80 mL) par une solution de NaOH à 4% dans l'eau (3 fois par 60 mL). Après évaporation des solvants sous pression réduite, le produit est précipité dans de l'éther de pétrole. Le précipité est filtré et séché pendant 15 à 20 heures sous pression atmosphérique à température ambiante.
Un solide blanc (8,55 g, rendement 44 %) de point de fusion 139 °C est obtenu.
La pureté molaire est supérieure à 94 % (RMN 1H).

Une caractérisation RMN ¹H et ¹³C est présentée dans le tableau 2 suivant.

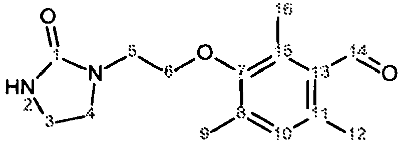

**Tableau 2**

| Atome | δ ¹H (ppm) + mult. | δ ¹³C (ppm) |
|---|---|---|
| 1 | - | 163.1 |
| 2 | ∼ 4.74 (s) | - |
| 3 | 3.40 (t) | 38.1 |
| 4 | 3.65 (t) | 46.8 |
| 5 | 3.52 (t) | 43.9 |
| 6 | 3.79 (t) | 71.3 |
| 7 | - | 153.9 |
| 8 | - | * |
| 9 | 2.23/2.46 (s) | 16.5/19.8 |
| 10 | 6.84 | 131.7 |
| 11 | - | * |
| 12 | 2.23/2.46 (s) | 16.5/19.8 |
| 13 | - | * |
| 14 | ∼ 10.46 (s) | 193.0 |
| 15 | - | * |
| 16 | 2.46 (s) | 12.1 |

| | | |
|---|---|---|
| * 131.4/133.5/136.6/136.7 ppm : Les déplacements chimiques des ¹³C du cycle aromatique ne sont pas attribués. | | |

Solvant utilisé : CDCl₃ - Calibration sur le signal du chloroforme à 7.2 ppm en ¹H, 77 ppm en ¹³C.

### e) Préparation du 2,4,6-trimethyl-3-(2-(2-oxoimidazolidin-1-yl)ethoxy)benzaldehyde oxime :

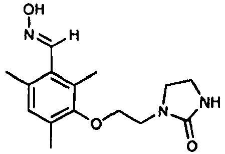

A une solution de 2,4,6-trimethyl-3-(2-(2-oxoimidazolidin-1-yl)ethoxy)benzaldehyde (7,90 g, 0,029 mol) dans l'éthanol (70 mL) maintenue à une température de 45°C est ajoutée une solution d'hydroxylamine aqueuse (2,83 g, 0,043 mol., 50 % dans l'eau) dans l'éthanol (10 mL). Le milieu réactionnel est ensuite agité pendant 2.5 heures à une température comprise entre 50 et 55°C. Le solvant est évaporé sous pression réduite (T_{bain} 37°C, 35 mbar). Au brut obtenu est ajouté de l'éther de pétrole (80 mL). Le précipité obtenu est filtré, lavé par de l'éther de pétrole (2 fois par 20 mL) et séché pendant 15 à 20 heures sous pression atmosphérique à température ambiante.
Un solide blanc (7,82 g, rendement 94 %) de point de fusion 165 °C est obtenu.
La pureté molaire est supérieure à 84 % (les 16 % restants comprennent notamment 7 % mol. d'EtOH) d'après la RMN ¹H.

Une caractérisation RMN ¹H et ¹³C est présentée dans le tableau 3 suivant.

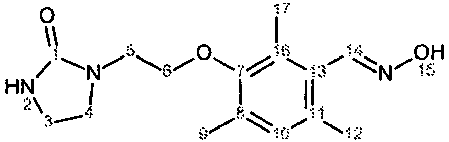

**Tableau 3**

| Atome | δ ¹H (ppm) + mult. | δ ¹³C (ppm) |
|---|---|---|
| 1 | - | 162.0 |
| 2 | ∼ 6.30 (s) | - |
| 3 | 3.19 (t) | 37.1 |
| 4 | 3.44 (t) | 45.5 |
| 5 | 3.34 (t) | 43.2 |
| 6 | 3.69 (t) | 70.3 |
| 7 | - | 153.5 |
| 8 | - | * |
| 9 | 2.14 (s) | 15.4 |
| 10 | - | 130.5 |
| 11 | - | * |
| 12 | 2.18 (s) | 19.9 |
| 13 | - | * |
| 14 | ∼ 8.20 (s) | 147.4 |
| 15 | ∼ 11.10 (s) | - |
| 16 | - | * |
| 17 | 2.17 (s) | 12.9 |

| | | |
|---|---|---|
| * 129.3/129.5/131.9 ppm : Les déplacements chimiques des ¹³C du cycle aromatique ne sont pas attribués, trois signaux sont détectés (probablement deux carbones sous un même signal). | | |

Solvant utilisé : DMSO - Calibration sur le signal du DMSO à 2.44 ppm en ¹H, 39.5 ppm en ¹³C.

### f) Préparation de 2,4,6-trimethyl-3-(2-(2-oxoimidazolidin-1-yl)ethoxy) nitriloxide, composé selon l'invention :

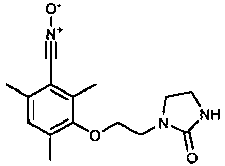

A une solution d'oxime précédemment préparée (6,00 g, 0,021 mol) dans le dichlorométhane (250 mL), à la température de 2°C, est ajoutée goutte à goutte une solution aqueuse de NaOCl (4 % de chlore actif, 52 mL) pendant 5-7 minutes. La température du milieu réactionnel est maintenue entre 0 et - 4°C. Le milieu réactionnel est ensuite agité pendant 3 heures à une température comprise entre 0 et 5°C. La phase organique est alors séparée. La phase aqueuse est extraite par du dichlorométhane (2 fois par 15 mL). Les phases organiques sont rassemblées puis lavées à l'eau (2 fois par 20 mL), séchées par Na₂SO₄. Le volume de solvant est réduit par évaporation sous pression réduite (T_{bain} 22 °C, 220 mbar) jusqu'à 50-60 mL. De l'éther de pétrole (75 mL) est alors ajouté et la solution est placée à -18°C pendant 10-15 heures. Le précipité obtenu est filtré et lavé par un mélange acétate d'éthyle / éther de pétrole (1/2) (10 mL) et enfin séché pendant 10-15 heures sous pression atmosphérique à température ambiante.
Un solide blanc (4,70 g, rendement 79 %) de point de fusion 156 °C est obtenu.
La pureté molaire est supérieure à 85 % (RMN ¹H).

Une caractérisation RMN ¹H et ¹³C est présentée dans le tableau 4 suivant.

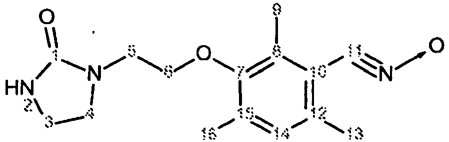

**Tableau 4**

| Atome | δ ¹H (ppm) + mult. | δ ¹³C (ppm) |
|---|---|---|
| 1 | - | Non détecté, non attribué |
| 2 | ∼ 4.59 (s) | - |
| 3 | 3.41 (t) | 38.3 |
| 4 | 3.64 (t) | 47.0 |
| 5 | 3.51 (t) | 44.1 |
| 6 | 3.79 (t) | 71.5 |
| 7 | - | 153.6 |
| 8 | - | 134.4/137.3* |
| 9 | 2.32 (s) | 14.8 |
| 10 | - | 112.8 |
| 11 | - | Non détecté, non attribué |
| 12 | - | 134.4/137.3* |
| 13 | 2.31 (s) | 20.2 |
| 14 | 6.85 (s) | 130.3 |
| 15 | - | 134.4/137.3* |
| 16 | 2.20 (s) | 16.4 |

| | | |
|---|---|---|
| * Les carbones aromatiques 8, 12 et 15 ne sont pas attribués. On observe deux signaux en RMN ¹³C, on a probablement deux carbones qui sortent sous le même signal. | | |

La fonction -C≡N→O présente une bande IR caractéristique à 2295 cm⁻¹

Solvant utilisé : CDCl₃ - Calibration sur le signal du chloroforme à 7.2 ppm en ¹H, 77 ppm en ¹³C.

### 1.2 - Greffage de l'agent de modification sur le SBR en masse

On utilise l'agent de modification que obtenu précédemment.
On incorpore du 2,4,6-trimethyl-3-(2-(2-oxoimidazolidin-1-yl)ethoxy) nitroxide (2,72g, 9,4 mmol), de pureté RMN de 86%mol, à 30g de SBR (contenant 26% en poids de styrène et 24% en poids d'unité butadiène-1,2 et de Mn = 162 900 g/mol et Ip = 1,15) sur outil à cylindres (mélangeur externe à 30°C). Le mélange est homogénéisé en 15 passes portefeuille.
Cette phase de mélangeage est suivie d'un traitement thermique sous presse à 10 bars de pression.
Les durées et les températures de cette seconde étape ont été modulées.
L'analyse par RMN ¹H a permis de déterminer le taux molaire de greffage et le rendement molaire de greffage qui sont reportés dans le tableau suivant:

**Tableau 5**

| **temps** | **T°** | **taux RMN (% mol)** | **rendement** |
|---|---|---|---|
| 5min | 110 | 1.55 | 90% |
| 10min | 110 | 1.55 | 90% |
| 5min | 150°C | 1.55 | 90% |
| 10min | 150°C | 1.54 | 90% |

### 1.3 - Greffage de l'agent de modification sur le SBR en solution

2 g de SBR (contenant 26% en poids de styrène et 24% en poids d'unité butadiène-1,2 et de Mn = 162 900 g/mol et Ip = 1,15) sont remis en solution dans 50 mL de dichlorométhane. Une solution de 60 mg (0.2 mmol) de 2,4,6-trimethyl-3-(2-(2-oxoimidazolidin-1-yl)ethoxy) nitriloxide dans 5 mL de dichlorométhane est ajoutée à la solution de polymère et le milieu réactionnel est agité pendant 24 h au reflux du dichlorométhane.
Le polymère est ensuite coagulé dans un mélange acétone/méthanol. Le polymère est remis en solution dans du toluène puis soumis à un traitement antioxydant par addition de 0,2 pce de 4,4'-methylène-bis-2,6-tert-butylphenol et 0,2 pce de N-(1,3-dimethylbutyl)-N'-phényl-p-phénylènediamine. Le polymère est séché sous vide pendant 48h à 60 °C.

L'analyse par RMN ¹H indique que le polymère a été modifié à hauteur de 1 % molaire ce qui équivaut encore à un rendement molaire de greffage de 67 %.

### Exemple 2 : Modification en masse d'un polyisoprène par greffage de 2,4,6-trimethyl-3-(2-(2-oxoimidazolidin-1-yl)ethoxy) nitriloxide

### 2.1 - Greffage de l'agent de modification sur le polyisoprène Natsyn 2200 (Goodyear)

On utilise le même agent de modification que celui précédemment obtenu dans l'exemple 1.
On incorpore 2.85g 2,4,6-trimethyl-3-(2-(2-oxoimidazolidin-1-yl)ethoxy) nitriloxide, de pureté RMN de 86%mol, à 30g de polyisoprène Natsyn 2200 ([ML(1+4) 100°C = 79, unités 3,4= 0.5%, unités trans 1,4= 1.9%, unité cis 1,4= 97,6%, Mw = 1044.10³ g/mol, Ip = 3.6]) sur outil à cylindres (mélangeur externe à 30°C). Le mélange est homogénéisé en 15 passes portefeuille.
Cette phase de mélangeage est suivie d'un traitement thermique sous presse à 10 bars de pression.
Les durées et les températures de cette seconde étape ont été modulées.
L'analyse par RMN ¹H a permis de déterminer le taux molaire de greffage et le rendement molaire de greffage qui sont reportés dans le tableau suivant:

**Tableau 6**

| temps | T° | taux RMN (% mol) | rdt |
|---|---|---|---|
| 5min | 130 | 1.52 | 88% |
| 10min | 130 | 1.55 | 90% |
| 5min | 150 | 1.54 | 90% |
| 10min | 150 | 1.58 | 92% |

### Exemple 3 : Modification d'un SBR par greffage de 3-methoxy-4-[2-(2-oxoimidazolidin-1-yl)ethoxy]benzonitrile oxide

### 1.1- Préparation de l'agent de modification:

### a) Préparation du 3-methoxy-4-[2-(2-oxoimidazolidin-1-yl)ethoxy]benzonitrile oxide

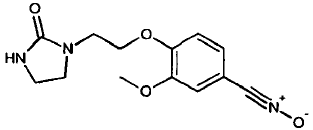

Ce composé peut être préparé à partir de la vanilline et de la 2-chloroethylimidazolidone selon le schéma synthétique suivant :

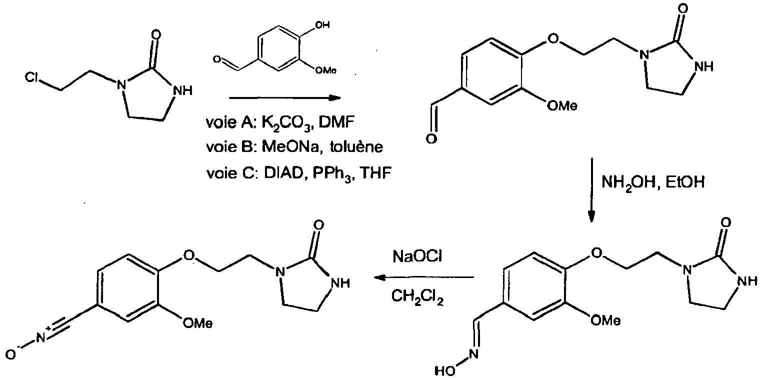

### b) Préparation du 3-methoxy-4-[2-(2-oxoimidazolidin-1-yl)ethoxy]benzaldehyde

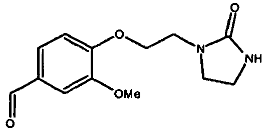

### Voie A

Une suspension de vanilline (30,0 g, 0,197 mol) et de K₂CO₃ (95,4 g, 0,690 mol) dans le DMF (200 mL) est portée à 50°C pendant 15 minutes. A cette suspension est ajouté par portions le 1-(2-chloroethyl)imidazolidin-2-one (44,0 g, 0,296 mol, pureté > 90%), dont la préparation a été décrite dans l'exemple 1, dans le DMF (30 mL). Le milieu réactionnel est chauffé à 90°C (T_{bain}) et cette température est maintenue pendant environ 4 heures. Le milieu réactionnel est ramené à température ambiante puis de l'eau (1,25 L) est ajoutée. Le produit est extrait par CH₂Cl₂ (400 mL, 4 fois par 100 mL). Les phases organiques rassemblées sont lavées par l'eau (60 mL) et concentrées sous pression réduite (14 mbar, 40°C). Le brut réactionnel est dilué par Et₂O (100 mL) et la suspension est agitée à température ambiante pendant 15-20 minutes. Le précipite obtenu est filtré, lavé par Et₂O (3 fois par 15 mL) et séché à température ambiante.
Un solide (31.2 g, rendement 60 %) de point de fusion 130 °C est obtenu.
La pureté molaire est supérieure à 92 % (RMN ¹H).

### Voie B

A une solution de sodium (1,51 g, 0,066 mol) dans CH₃OH (60 mL) est ajoutée de la vanilline (10,0 g, 0,066 mol) dans le toluène anhydre (250 mL). Le milieu réactionnel, sous atmosphère inerte, est porté au reflux puis le méthanol résiduel est distillé. Après retour à 80-90 °C, une suspension de 1-(2-chloroethyl)imidazolidin-2-one (9,28 g, 0,064 mol, pureté > 95%) dans le toluène (50 mL) est ajoutée en une fois au milieu réactionnel. Après 25 heures de réaction, le milieu réactionnel est concentré sous pression réduite (T_{bain} 50°C, 30 mbar). Le brut réactionnel est repris dans CH₂Cl₂ (150 mL). La vanilline non réagie est éliminée par extraction avec une solution aqueuse de 7 % NaOH (5 fois par 30 mL). Les phases organiques rassemblées sont lavées par de l'eau (4 fois 50 mL), séchées sous Na₂SO₄ et évaporées sous pression réduite (T_{bain} 27 °C, 20 mbar). Le brut réactionnel (4,81 g) est dilué par un mélange d'éther de pétrole et EtOAc et le précipité obtenu est filtré.
Un solide (0,91 g, rendement 6 %) de point de fusion 127 °C est obtenu.
La pureté molaire est supérieure à 81 % (RMN ¹H).

### Voie C

Le mode opératoire de la réaction de Mitsunobu est par exemple décrit dans les références suivantes : Mitsunobu, O.; Yamada, Y. Bull. Chem. Soc. Japan 1967, 40, 2380-2382, The Use of Diethyl Azodicarboxylate and Triphenylphosphine in Synthesis and Transformation of Nataural Products Mitsunobu, O. Synthesis 1981, 1-28, brevet EP1149092 B1, 2003.

A une solution de vanilline (5,02 g, 0,033 mol), de 1-(2-hydroxyethyl)imidazolidin-2-one anhydre (6,38 g, 0,049 mol, Aldrich) et de PPh₃ (13,1 g, 0,050 mol) dans le THF anhydre (300 mL) à 2°C est ajouté goutte à goutte, sur 20 minutes, une solution de diisopropyl azodicarboxylate (10,1 g, 0,050 mol, Aldrich) dans le THF anhydre (150 mL). Le milieu réactionnel est agité pendant 14 heures à température ambiante puis est dilué par l'eau (150 mL). Le milieu réactionnel est concentré sous pression réduite (45 mbar, T_{bain} 28°C). La phase aqueuse est extraite par EtOAc (3 fois par 200 mL). Les phases organiques rassemblées sont lavées par une solution aqueuse de NaCl saturée puis sont concentrées sous pression réduite de façon à obtenir une solution de 150 mL. Le brut réactionnel en solution est purifié par chromatographie sur colonne (SiO₂, éluant 1 : EtOAc, éluant 2 : EtOAc/EtOH = 4/1, Rf du produit 0,36, Rf de Ph₃PO 0,71 dans EtOAc : EtOH = 5:1).
Un solide (6,59 g, rendement 76 %) de point de fusion 130 °C est obtenu.
La pureté molaire est supérieure à 88 % (RMN ¹H).
Le 3-methoxy-4-[2-(2-oxoimidazolidin-1-yl)ethoxy]benzaldehyde obtenu est directement engagé dans l'étape suivante sans purification supplémentaire.

### Caractérisation RMN ¹H et ¹³_{C}

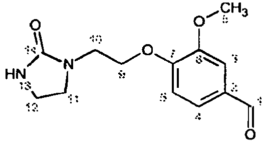

**Tableau 7**

| **N° atomes** | **δ du ¹H (ppm)** | **δ du ¹³C (ppm)** |
|---|---|---|
| 1 | 9,78 | 191,1 |
| 2 | / | 129,6 |
| 3 | 7,34 | 109,6 |
| 4 | 7,48 | 125,6 |
| 5 | 7,14 | 112,0 |
| 6 | / | 148,9 |
| 7 | / | 152,9 |
| 8 | 3,78 | 55,4 |
| 9 | 4,11 | 67,3 |
| 10 | 3,42 | 45,5 |
| 11 | 3,38 | 42,3 |
| 12 | 3,16 | 37,2 |
| 13 | 6,33 | / |
| 14 | / | 161,9 |

Solvant utilisé : DMSO - Calibration sur le signal du DMSO à 2.44 ppm en ¹H, 39.5 ppm en ¹³C.

### c) Préparation 3-methoxy-4-[2-(2-oxoimidazolidin-1-yl)ethoxy]benzaldehyde oxime :

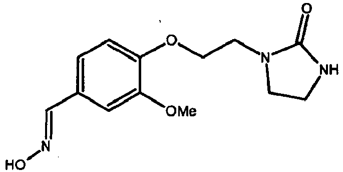

A une solution de 3-methoxy-4-[2-(2-oxoimidazolidin-1-yl)ethoxy]benzaldehyde (25,6 g, 0,097 mol) dans EtOH (250 mL) à 52 °C est ajoutée une solution d'hydroxylamine (10,2 g, 0,155 mol, 50 % dans l'eau, Aldrich) dans EtOH (20 mL). Le milieu réactionnel est ensuite agité pendant 4,5 heures entre 50 et 60°C. Le milieu réactionnel est ensuite concentré sous pression réduite (T_{bain} = 42 °C, 60 mbar) de façon à obtenir un résidu de 70-80 mL. Le précipité obtenu est filtré, lavé par un mélange EtOH/eau (2 fois 5 mL/15 mL) et séché sous pression atmosphérique à température ambiante.
Un solide blanc (22,14 g, rendement 82 %) de point de fusion 189 °C est obtenu.
La pureté molaire est supérieure à 89 % (RMN ¹H).

### Caractérisation RMN ¹H et ¹³C

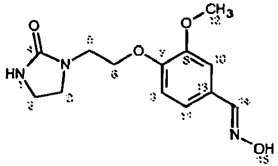

**Tableau 8**

| **N° atomes** | **δ du ¹H (ppm)** | **δ du ¹³C (ppm)** |
|---|---|---|
| 1 | 6,30 | / |
| 2 | 3,16 | 37,1 |
| 3 | 3,35 | 42,4 |
| 4 | / | 161,9 |
| 5 | 3,42 | 45,4 |
| 6 | 4,00 | 67,0 |
| 7 | / | 148,5 |
| 8 | / | 148,9 |
| 9 | 6,93 | 112,8 |
| 10 | 7,15 | 108,6 |
| 11 | 7,01 | 119,9 |
| 12 | 3,72 | 55,2 |
| 13 | / | 125,9 |
| 14 | 7,98 | 147,5 |
| 15 | 10,92 | / |

Solvant utilisé : DMSO - Calibration sur le signal du DMSO à 2.44 ppm en ¹H, 39.5 ppm en ¹³C.

### d) Préparation du 3-methoxy-4-[2-(2-oxoimidazolidin-1-yl)ethoxy]benzonitrile oxide

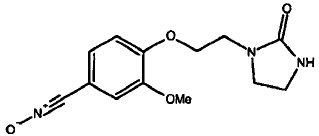

A une suspension de 3-methoxy-4-[2-(2-oxoimidazolidin-1-yl)ethoxy]benzaldehyde oxime (21,7 g, 0,078 mol) dans CH₂Cl₂ (950 mL) à -3°C, est ajoutée, goutte à goutte, une solution aqueuse de NaOCl dans l'eau (Aldrich, > 4 % de chlore actif) (161 mL) pendant 10 minutes. Le milieu réactionnel est ensuite agité 20 minutes à 0°C. La phase organique est séparée et la phase aqueuse est extraite par CH₂Cl₂ (4 fois par 100 mL). Les phases organiques rassemblées sont lavées par l'eau (3 fois par 100 mL), séchées sur Na₂SO₄ puis concentrées sous pression réduite (T_{bain} 22°C) jusqu'à 200-220 mL. Le précipité obtenu est filtré, lavé par CH₂Cl₂ (2 fois par 10 mL) et séché sous pression atmosphérique à température ambiante.
Un solide (9,13 g, rendement 42 %) de point de fusion 109-111 °C avec dégradation est obtenu.
La pureté molaire est supérieure à 80 % (RMN ¹H). Par recristallisation dans EtOH, la pureté du composé est supérieure à 90% massique

### Caractérisation RMN ¹H et ¹³C

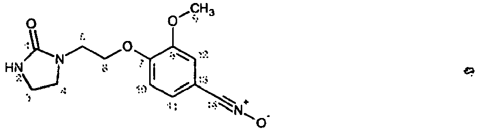

**Tableau 9**

| **N° atomes** | **δ du ¹H (ppm)** | **δ du ¹³C (ppm)** |
|---|---|---|
| 1 | / | 163,5 |
| 2 | 6,31 | / |
| 3 | 3,15 | 37,2 |
| 4 | 3,35 | 42,3 |
| 5 | 3,40 | 45,4 |
| 6 | 4,04 | 67,1 |
| 7 | / | 150,4 |
| 8 | / | 148,4 |
| 9 | 3,73 | 55,6 |
| 10 | 7,03 | 113,0 |
| 11 | 7,25 | 125,8 |
| 12 | 7,32 | 115,2 |
| 13 | / | 106,2 |
| 14 | / | Non visible |

Solvant utilisé : DMSO - Calibration sur le signal du DMSO à 2.44 ppm en ¹H, 39.5 ppm en ¹³C.

### Caractérisation Infra-Rouge (pastille KBr)

v (cm⁻¹) : 2305 (fonction Ar-C≡N→O)

### Caractérisation par spectrométrie de masse

C₁₃H₁₅N₃O₄, Mw= 277.27 g/mol

Les échantillons ont été analysés par introduction directe dans le spectromètre de masse, en utilisant le mode d'ionisation par électrospray (ID/ESI).

### Préparation de l'échantillon :

Environ 20 mg d'échantillon a été mis en solution dans 25 ml de méthanol puis dilué au 1/100 pour l'analyse par ID/ESI.

### Mode positif :

*m*/*z :* 300 ([[M+Na]⁺), 577 ([2M+Na]⁺)

### 1.2 - Greffage de l'agent de modification sur le SBR en masse

On utilise l'agent de modification obtenu précédemment.
On incorpore du 3-methoxy-4-[2-(2-oxoimidazolidin-1-yl)ethoxy]benzonitrile oxide (1.24g, 4,5 mmol), de pureté RMN de 90%mol, à 30g de SBR (contenant 26% en poids de styrène et 24% en poids d'unité butadiène-1,2 et de Mn = 162 900 g/mol et Ip = 1,15)sur outil à cylindres (mélangeur externe à 30°C).). Le mélange est homogénéisé en 15 passes portefeuille.
Cette phase de mélangeage est suivie d'un traitement thermique (10min à 110°C) sous presse à 10 bars de pression.
L'analyse par RMN ¹H a permis de déterminer le taux molaire de greffage (0.78%mol) et le rendement molaire de greffage (87%).

### Exemple 4 : Modification d'un SBR par greffage du 2-[2-(2-oxoimidazolidin-1-yl)ethoxylbenzonitrile oxide

### 1.1 - Préparation de l'agent de modification:

### a) Préparation du 2-[2-(2-oxoimidazolidin-1-yl)ethoxylbenzonitrile oxide

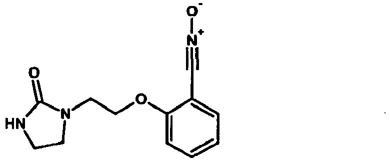

Ce composé peut être préparé à partir de l'aldéhyde salicylique et de la 2-chloroethylimidazolidone selon le schéma synthétique suivant :

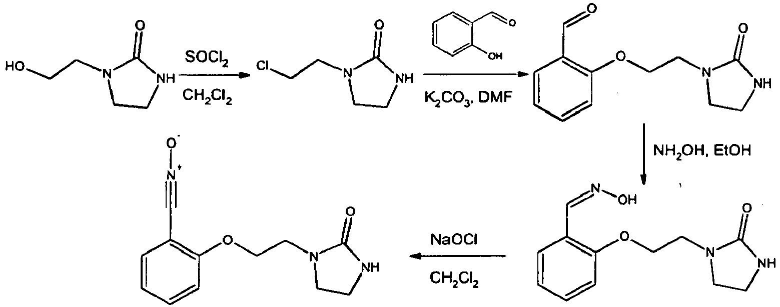

### b) Préparation du 2-[2-(2-oxoimidazolidin-1-yl)ethoxy]benzaldehyde

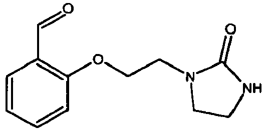

A une solution d'aldéhyde salicylique (22.0g, 0.180 mol) dans le DMF (100 mL) est ajouté du K₂CO₃ (87.1g, 0.631 mol). Le mélange est agité à 52°C. Après 10 minutes à cette température, est ajouté par portions le 1-(2-chloroethyl)imidazolidin-2-one (40,0 g, 0,270 mol, pureté > 90 %) dont la préparation a été décrite dans l'exemple 1. La température du mélange est amenée à 90°C (T_{bain}) en une heure et cette température est maintenue pendant 5 heures. Après retour à température ambiante, le mélange est dilué par de l'eau (1,3 L) et le produit est extrait par CH₂Cl₂ (500 mL, 5 fois 100 mL). Les phases organiques sont rassemblées, puis lavées par l'eau (deux fois par 50 mL) et évaporées jusqu'à obtention d'un brut réactionnel de 70-80 g (suspension dense) (T_{bain} = 40°C). Le brut réactionnel est repris dans Et₂O (120 mL) et la suspension est agitée à température ambiante pendant 20 minutes. Le précipité obtenu est filtré et lavé par un mélange de DMF/Et₂O/H₂O (5mL/20mL/15mL) puis par Et₂O (2 fois par 10 mL). Le solide obtenu est séché à température ambiante.
Un solide (30,6 g, rendement 73 %) de point de fusion 150 °C est obtenu. La pureté molaire est supérieure à 84 % (RMN ¹H).
Le 2-[2-(2-oxoimidazolidin-1-yl)ethoxy]benzaldehyde obtenu est directement engagé dans l'étape suivante sans purification supplémentaire.

### Caractérisation RMN ¹H et ¹³C

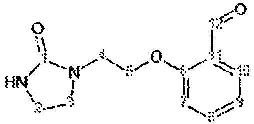

**Tableau 10**

| Atome | δ ¹H (ppm) | δ ¹³C (ppm) |
|---|---|---|
| 1 | - | 164.9 |
| 2 | 3.15 | 37.3 |
| 3 | 3.39 | 44.9 |
| 4 | 3.44 | 42.1 |
| 5 | 4.16 | 66.5 |
| 6 | - | 160.5 |
| 7 | 7.17 | 113.2 |
| 8 | 7.59 | 136.2 |
| 9 | 7.02 | 120.5 |
| 10 | 7.63 | 127.3 |
| 11 | - | 124.0 |
| 12 | 10.31 | 189.1 |

Solvant utilisé : DMSO - Calibration sur le signal du DMSO à 2.44 ppm en ¹H, 39.5 ppm en ¹³C.

### c) Préparation du 2-[2-(2-oxoimidazolidin-1-yl)ethoxy]benzaldehyde oxime

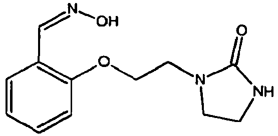

Une solution du 2-[2-(2-oxoimidazolidin-1-yl)ethoxy]benzaldehyde (10,0 g, 0,043 mol) dans EtOH (100 mL) est portée à 50°C. A cette température, une solution d'hydroxylamine (4,5 g, 0,068 mol, 50 % dans l'eau, Aldrich) dans EtOH (10 mL) est ajoutée. Le milieu réactionnel est ensuite agité pendant 6 heures à une température comprise entre 50°C et 70°C. Le milieu réactionnel est évaporé sous pression réduite (T_{bain} 45 °C, 65-70 mbar) jusqu'à l'obtention d'une suspension. Le brut réactionnel est ensuite repris dans l'eau (5 mL). La solution obtenue est refroidie à 5°C et maintenue à cette température pendant 15 heures. Le précipité obtenu est filtré et lavé sur le filtre par un coupage EtOH/eau (2 mL/2 mL) puis par un coupage EtOH/éther de pétrole (1 mL / 4 mL) puis par de l'éther de pétrole (2*10 mL). Le solide est alors séché sous pression atmosphérique à température ambiante.
Un solide blanc (9,25 g, rendement 87%) de point de fusion 88°C est obtenu.
La pureté molaire est supérieure à 99% (RMN ¹H).

### Caractérisation RMN ¹H et ¹³C

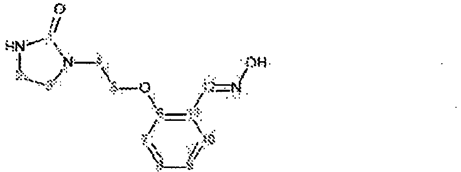

**Tableau 11**

| Atome | ¹H (ppm) | δ ¹³C (ppm) |
|---|---|---|
| 1 | - | 162.0 |
| 2 | 3.17 | 37.4 |
| 3 | 3.37 | 45.4 |
| 4 | 3.39 | 42.6 |
| 5 | 4.03 | 66.6 |
| 6 | - | 155.8 |
| 7 | 7.01 | 113.0 |
| 8 | 7.28 | 130.7 |
| 9 | 6.89 | 121.2 |
| 10 | 7.61 | 125.2 |
| 11 | - | 120.9 |
| 12 | 8.25 | 143.3 |

Solvant utilisé : DMSO - Calibration sur le signal du DMSO à 2.44 ppm en ¹H, 39.5 ppm en ¹³C.

### d) Préparation du 2-[2-(2-oxoimidazolidin-1-yl)ethoxy]benzonitrile oxide

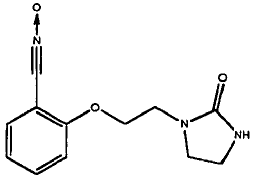

A une suspension de 2-[2-(2-oxoimidazolidin-1-yl)ethoxy]benzaldehyde oxime (20,2 g, 0,081 mol) dans CH₂Cl₂ (400 mL) à -1°C est ajoutée, goutte à goutte pendant 10 minutes, une solution aqueuse de NaOCl dans l'eau (157 mL, Aldrich, > 4 % de chlore actif). Le milieu réactionnel est ensuite agité pendant 20 minutes. Les phases aqueuses et organiques sont séparées et la phase aqueuse est extraite par CH₂Cl₂ (2 fois 75 mL). Les phases organiques rassemblées sont lavées par l'eau (3 fois 10 mL) et séchées sur Na₂SO₄. Les phases sont concentrées à 100mL sous pression réduite à température ambiante. 50 mL d'éther de pétrole sont ajoutés. La solution est refroidie jusqu'à -18°C (3 heures). Le précipité est filtré, lavé par CH₂Cl₂ / éther de pétrole (5 mL/ 10 mL ; puis 5 mL/ 20 mL; puis 0 mL/ 20 mL) puis séché sous pression atmosphérique à température ambiante.
Un solide (11,32 g, rendement 57 %) de point de fusion 109-110 °C avec dégradation du produit est obtenu.
La pureté molaire est supérieure à 94 % (RMN ¹H).

### Caractérisation RMN ¹H et ¹³C

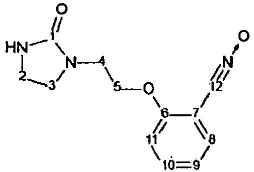

**Tableau 12**

| Atome | δ ¹H (ppm) | δ ¹³C (ppm) |
|---|---|---|
| 1 | - | 162.0 |
| 2 | 3.18 | 37.5 |
| 3 | 3.45 | 45.8 |
| 4 | 3.39 | 42.5 |
| 5 | 4.14 | 67.9 |
| 6 | - | 159.9 |
| 7 | - | 101.6 |
| 8 | 7.60 | 133.4 |
| 9 | 7.00 | 121.2 |
| 10 | 7.48 | 132.9 |
| 11 | 7.16 | 112.6 |
| 12 | - | Non observé |
| NH | ∼ 6.34 | - |

Solvant utilisé : DMSO - Calibration sur le signal du DMSO à 2.44 ppm en ¹H, 39.5 ppm en ¹³C.

### Caractérisation Infra-Rouge (pastille KBr)

*v* (cm⁻¹) : 2295 (fonction Ar-C≡N→O)

### Caractérisation par spectrométrie de masse

C₁₂H₁₃N₃O₃, Mw= 247.25 g/mol

Les échantillons ont été analysés par introduction directe dans le spectromètre de masse, en utilisant le mode d'ionisation par électrospray (ID/ESI).

### Préparation de l'échantillon

20 mg de l'échantillon est dissous dans 2 mL d'acétonitrile

*m*/*z :* 270 ([[M+Na]⁺), 517 ([2M+Na]⁺)

### 1.2 - Greffage de l'agent de modification sur le SBR en masse

On utilise l'agent de modification obtenu précédemment.
On incorpore du 2-[2-(2-oxoimidazolidin-1-yl)ethoxy]benzonitrile oxide (0.55g, 2.24 mmol), de pureté RMN de 94%mol, à 30g de SBR (contenant 26% en poids de styrène et 24% en poids d'unité butadiène-1,2 et de Mn = 162 900 g/mol et Ip = 1,15) sur outil à cylindres (mélangeur externe à 30°C). Le mélange est homogénéisé en 15 passes portefeuille.
Cette phase de mélangeage est suivie d'un traitement thermique (10min à 110°C) sous presse à 10 bars de pression.
L'analyse par RMN ¹H a permis de déterminer le taux molaire de greffage (0.34%mol) et le rendement molaire de greffage (71%).

## Revendications

1. Polymère modifié obtenu par greffage d'un composé comprenant au moins un groupement Q, et au moins un groupement A reliés entre eux par au moins et de préférence un groupement « espaceur » Sp dans lequel :
- Q comprend un dipôle contenant au moins et de préférence un atome d'azote,
- A comprend un groupe associatif comprenant au moins un atome d'azote,
- Sp est un atome ou un groupe d'atomes formant une liaison entre Q et A,
le groupe associatif étant choisi parmi un groupe imidazolidinyle, triazolyle, uréyle, bis-uréyle, uréido-pyrimidyle.

2. Polymère modifié selon la revendication 1, **caractérisé en ce que** le polymère est un élastomère diénique.

3. Polymère modifié selon la revendication 1 ou 2, **caractérisé en ce que** l'élastomère diénique est essentiellement saturé, de préférence choisi parmi les copolymères d'éthylène-propylène-monomère diène (EPDM), les caoutchoucs butyle.

4. Polymère modifié selon la revendication 1 ou 2, **caractérisé en ce que** l'élastomère diénique est essentiellement insaturé, de préférence choisi parmi le caoutchouc naturel, les polyisoprènes de synthèse, les polybutadiènes, les copolymères de butadiène, les copolymère d'isoprène et les mélanges de ces élastomères.

5. Polymère modifié selon l'une quelconque des revendications 1 à 4, **caractérisé en ce que** le groupement A répond à l'une des formules (II) à (VI) suivantes : 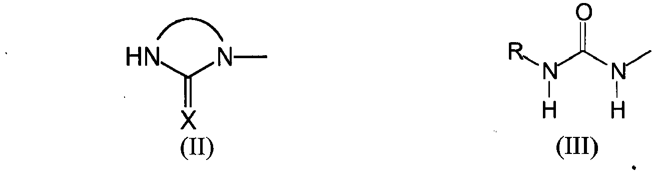 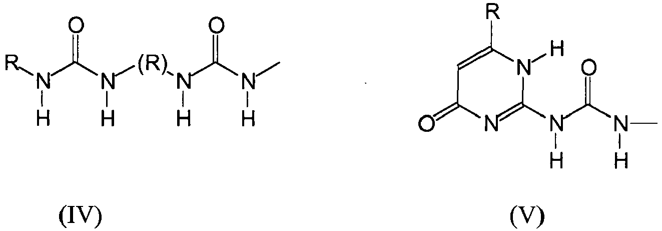 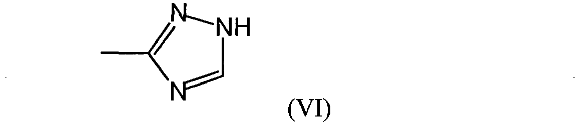 où :
- R désigne un groupement hydrocarboné pouvant éventuellement contenir des hétéroatomes,
- X désigne un atome d'oxygène, de soufre ou un groupement -NH de préférence un atome d'oxygène.

6. Polymère modifié selon l'une quelconque des revendications 1 à 5 **caractérisé en ce que** le groupement Q comprend une fonction oxyde de nitrile, nitrone, ou nitrile imine.

7. Polymère modifié selon l'une quelconque des revendications 1 à 6 **caractérisé en ce que** le groupement Q est un groupe de formule (VII), (VIII) ou (IX) suivante 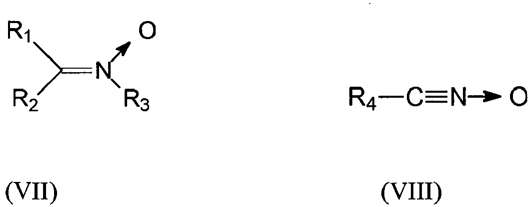 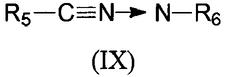 dans lesquelles R1 à R6 sont choisis indépendamment parmi un groupe espaceur Sp, un atome d'hydrogène, un groupe alkyle en C1-C20 linéaire ou ramifié, un groupe cycloalkyle en C3-C20 linéaire ou ramifié, un groupe aryle en C6-C20 linéaire ou ramifié, et un groupe de formule (X) 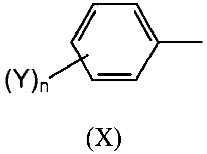 dans laquelle n représente 1, 2, 3, 4 ou 5 et chaque Y représente indépendamment un groupe espaceur Sp, un groupe alkyle ou un halogénure.

8. Polymère modifié selon l'une quelconque des revendications 1 à 7 **caractérisé en ce que** le groupement « espaceur » est une chaîne alkyle linéaire ou ramifiée en C1-C24, de préférence C1-C10 comprenant un ou plusieurs hétéroatomes choisis parmi les atomes d'azote, de soufre, de silicium ou d'oxygène.

9. Polymère modifié selon l'une quelconque des revendications 1 à 8 **caractérisé en ce que** le groupement Q est un groupement de formule (XIa) ou (XIb): 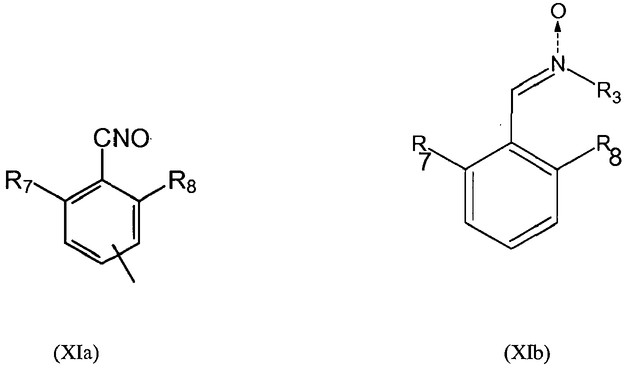 dans laquelle R7 et R8 représentent indépendamment un groupe alkyle en C1-C5 ou un halogénure et de préférence R7 et R8 représentent indépendamment un groupe méthyle ou un atome de chlore, R3 est tel que défini dans la revendication 8 et le groupement A est un groupement de formule (XII)

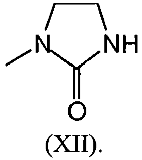

10. Polymère modifié selon l'une quelconque des revendications 1 à 9 **caractérisé en ce qu'**il est choisi parmi les composés de formule (XIII) à (XXI) suivantes: 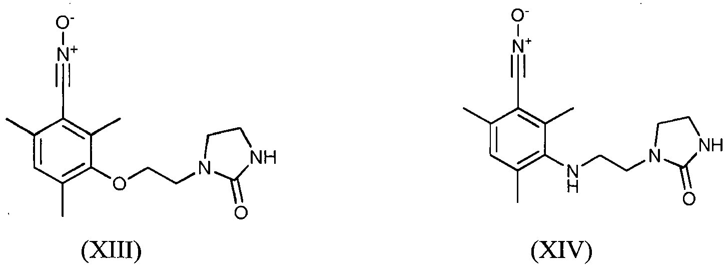 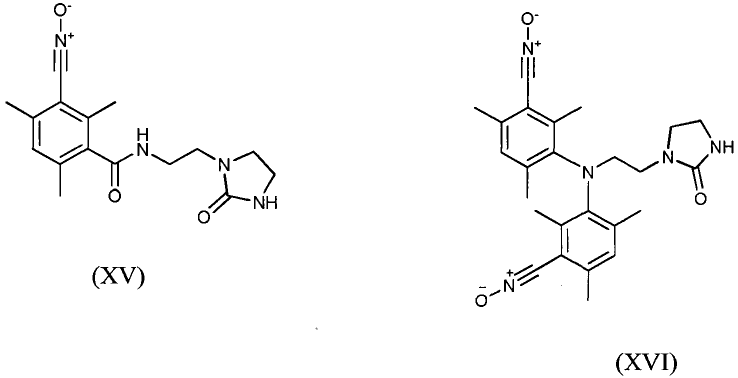 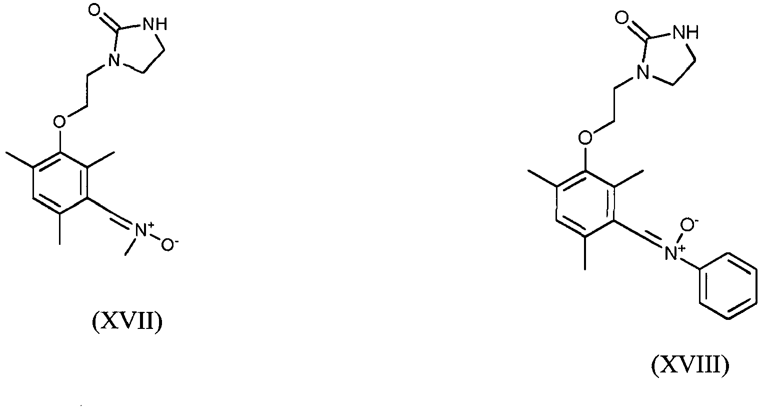 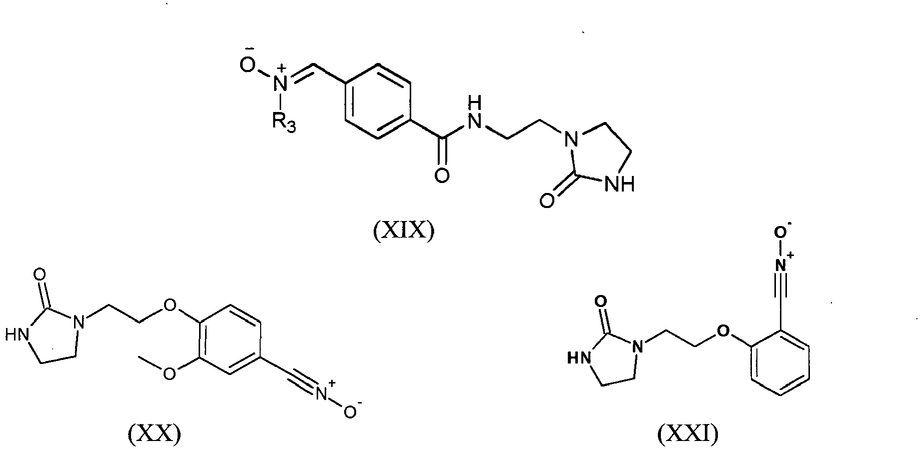 R3 étant tel que défini dans la revendication 7

11. Polymère modifié selon l'une quelconque des revendications 1 à 8 **caractérisé en ce qu'**il est choisi parmi les composés de formule (XXII) et (XXIII) suivantes: 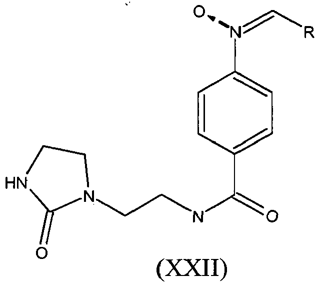 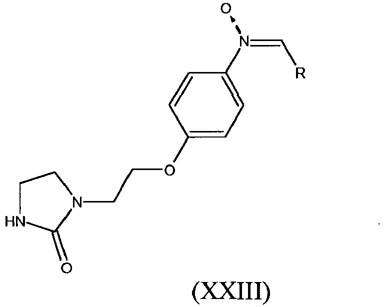 dans lesquelles R est choisi indépendamment parmi un groupe espaceur Sp, un atome d'hydrogène, un groupe alkyle en C1-C20 linéaire ou ramifié, un groupe cycloalkyle en C3-C20 linéaire ou ramifié, un groupe aryle en C6-C20 linéaire ou ramifié, et un groupe de formule (X) 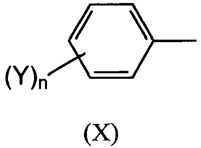 dans laquelle n représente 1, 2, 3, 4 ou 5 et chaque Y représente indépendamment un groupe espaceur Sp, un groupe alkyle ou un halogénure.

12. Procédé de préparation d'un polymère modifié comprenant une étape de greffage sur un polymère comportant au moins une insaturation d'un composé comprenant au moins un groupement Q, et au moins un groupement A reliés entre eux par au moins et de préférence un groupement « espaceur » Sp tels que défini dans les revendications 1 à 11, par cycloaddition [3+2] du groupement Q sur ladite insaturation.

13. Procédé de préparation d'un polymère modifié selon la revendication 12 **caractérisé en ce que** le polymère est un élastomère diénique.

## Patentansprüche

1. Modifiziertes Polymer, erhalten durch Pfropfen einer Verbindung mit mindestens einer Gruppe Q und mindestens einer Gruppe A, die über mindestens und vorzugsweise eine Spacergruppe Sp miteinander verbunden sind, wobei:
- Q einen Dipol, der mindestens und vorzugsweise ein Stickstoffatom umfasst,
- A eine assoziative Gruppe mit mindestens einem Stickstoffatom umfasst,
- Sp ein Atom oder eine Gruppe von Atomen, das bzw. die eine Bindung zwischen Q und A bildet bzw. bilden,
wobei die assoziative Gruppe aus einer Imidazolidinyl-, Triazolyl-, Ureyl-, Bisureyl- oder Ureidopyrimidylgruppe ausgewählt ist.

2. Modifiziertes Polymer nach Anspruch 1, **dadurch gekennzeichnet, dass** es sich bei dem Polymer um ein Dienelastomer handelt.

3. Modifiziertes Polymer nach Anspruch 1 oder 2, **dadurch gekennzeichnet, dass** das Dienelastomer im Wesentlichen gesättigt ist und vorzugsweise aus Ethylen-Propylen-Dienmonomer-Copolymeren (EPDM-Copolymeren) und Butylkautschuken ausgewählt ist.

4. Modifiziertes Polymer nach Anspruch 1 oder 2, **dadurch gekennzeichnet, dass** das Dienelastomer im Wesentlichen ungesättigt ist und vorzugsweise aus Naturkautschuk, synthetischen Polyisoprenen, Polybutadienen, Butadien-Copolymeren, Isopren-Copolymeren und Mischungen dieser Elastomere ausgewählt ist.

5. Modifiziertes Polymer nach einem der Ansprüche 1 bis 4, **dadurch gekennzeichnet, dass** die Gruppe A einer der folgenden Formeln (II) bis (VI) entspricht: 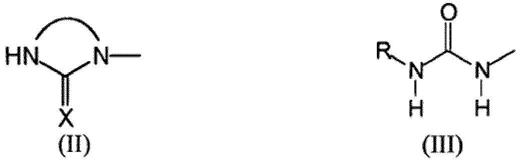 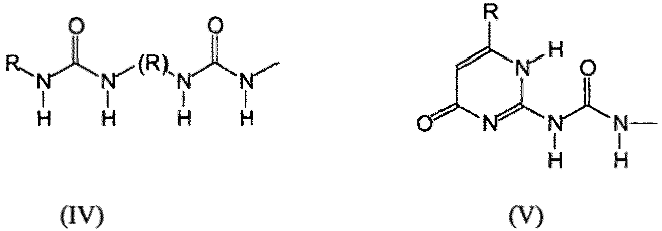 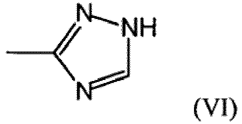 wobei:
- R für eine Kohlenwasserstoffgruppe, die gegebenenfalls Heteroatome enthalten kann, steht,
- X für ein Sauerstoff- oder Schwefelatom oder eine Gruppe -NH, vorzugsweise ein Sauerstoffatom, steht.

6. Modifiziertes Polymer nach einem der Ansprüche 1 bis 5, **dadurch gekennzeichnet, dass** die Gruppe Q eine Nitriloxid-, Nitron- oder Nitriliminfunktion umfasst.

7. Modifiziertes Polymer nach einem der Ansprüche 1 bis 6, **dadurch gekennzeichnet, dass** es sich bei der Gruppe Q um eine Gruppe der folgenden Formeln (VII), (VIII) oder (IX) handelt: 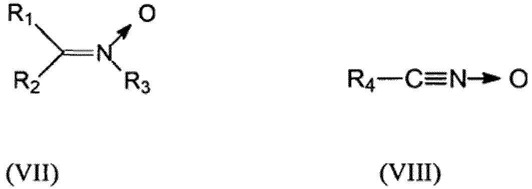 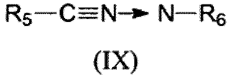 worin R1 bis R6 unabhängig aus einer Spacergruppe Sp, einem Wasserstoffatom, einer linearen oder verzweigten C1-C20-Alkylgruppe, einer linearen oder verzweigten C3-C20-Cycloalkylgruppe, einer linearen oder verzweigten C6-C20-Arylgruppe und einer Gruppe der Formel (X) 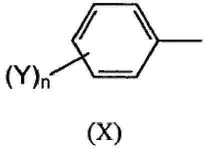 worin n für 1, 2, 3, 4 oder 5 steht und Y jeweils unabhängig für eine Spacergruppe Sp, eine Alkylgruppe oder ein Halogenid steht, ausgewählt sind.

8. Modifiziertes Polymer nach einem der Ansprüche 1 bis 7, **dadurch gekennzeichnet, dass** es sich bei der Spacergruppe um eine lineare oder verzweigte C1-C24-Alkylkette, vorzugsweise C1-C10-Alkylkette, mit einem oder mehreren Heteroatomen, die aus Stickstoff-, Schwefel-, Silicium- oder Sauerstoffatomen ausgewählt sind, handelt.

9. Modifiziertes Polymer nach einem der Ansprüche 1 bis 8, **dadurch gekennzeichnet, dass** es sich bei der Gruppe Q um eine Gruppe der Formel (XIa) oder (XIb) handelt: 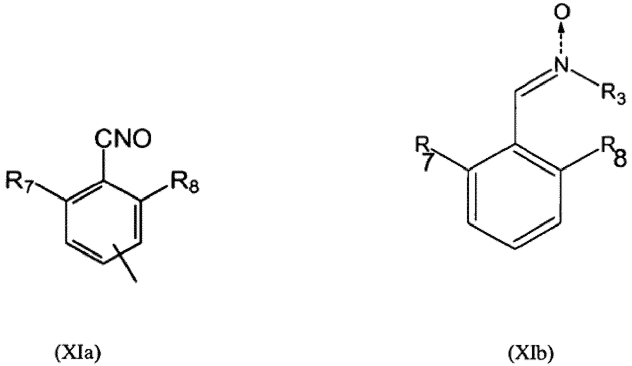 worin R7 und R8 unabhängig für eine C1-C5-Alkylgruppe oder ein Halogenid stehen und R7 und R8 vorzugsweise unabhängig für eine Methylgruppe oder ein Chloratom stehen, R3 wie in Anspruch 8 definiert ist und es sich bei der Gruppe A um eine Gruppe der Formel (XII) 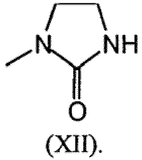 handelt.

10. Modifiziertes Polymer nach einem der Ansprüche 1 bis 9, **dadurch gekennzeichnet, dass** es aus den Verbindungen der folgenden Formeln (XIII) bis (XXI) ausgewählt ist: 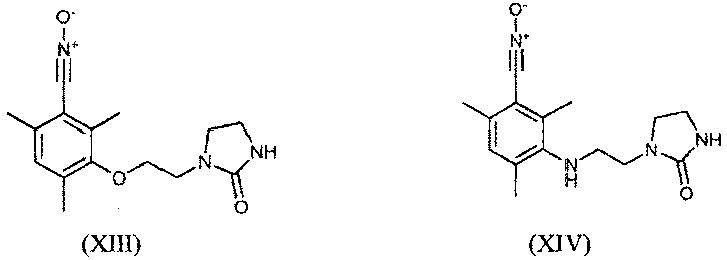 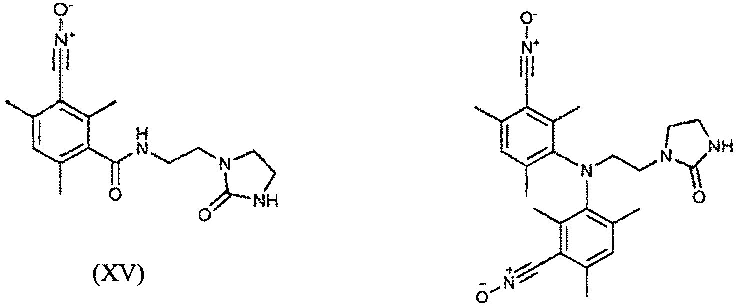 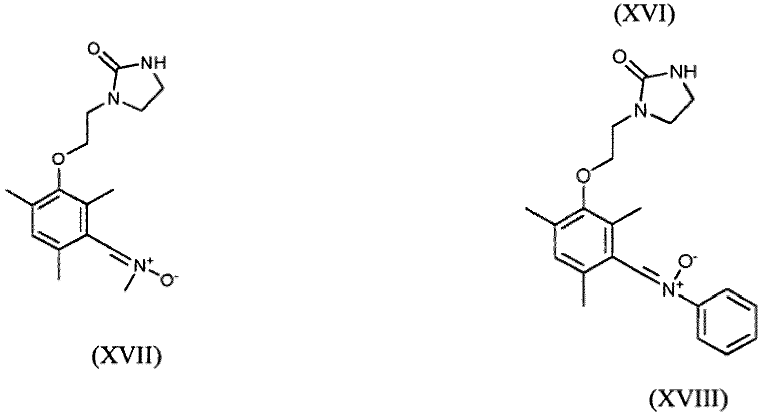 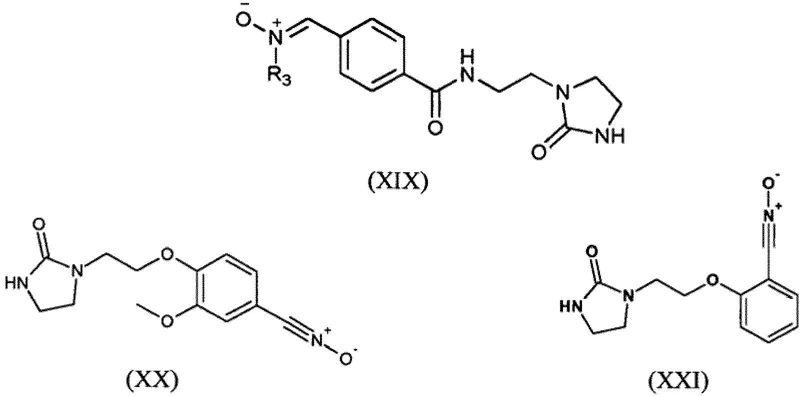 wobei R3 wie in Anspruch 7 definiert ist.

11. Modifiziertes Polymer nach einem der Ansprüche 1 bis 8, **dadurch gekennzeichnet, dass** es aus den Verbindungen der folgenden Formeln (XXII) und (XXIII) ausgewählt ist: 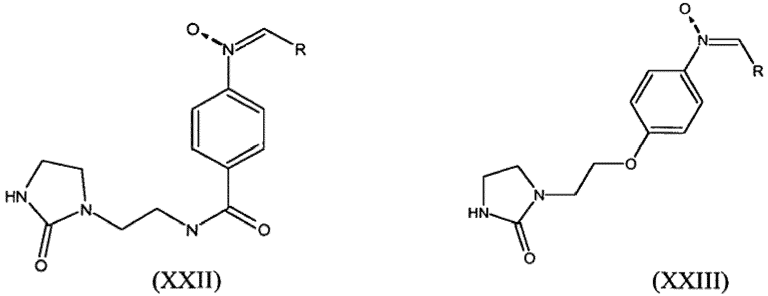 worin R unabhängig aus einer Spacergruppe Sp, einem Wasserstoffatom, einer linearen oder verzweigten C1-C20-Alkylgruppe, einer linearen oder verzweigten C3-C20-Cycloalkylgruppe, einer linearen oder verzweigten C6-C20-Arylgruppe und einer Gruppe der Formel (X) 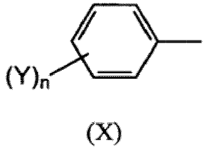 worin n für 1, 2, 3, 4 oder 5 steht und Y jeweils unabhängig für eine Spacergruppe Sp, eine Alkylgruppe oder ein Halogenid steht, ausgewählt ist.

12. Verfahren zur Herstellung eines modifizierten Polymers, bei dem man auf ein Polymer mit mindestens einer Ungesättigtheit eine Verbindung mit mindestens einer Gruppe Q und mindestens einer Gruppe A, die über mindestens und vorzugsweise eine Spacergruppe Sp miteinander verbunden sind, gemäß einem der Ansprüche 1 bis 11 durch [3+2]-Cycloaddition der Gruppe Q an die Ungesättigtheit aufpfropft.

13. Verfahren zur Herstellung eines modifizierten Polymers nach Anspruch 12, **dadurch gekennzeichnet, dass** es sich bei dem Polymer um ein Dienelastomer handelt.

## Claims

1. Modified polymer obtained by grafting of a compound comprising at least one group Q and at least one group A, bonded together by at least, and preferably, one "spacer" group Sp in which:
- Q comprises a dipole containing at least, and preferably, one nitrogen atom,
- A comprises an associative group comprising at least one nitrogen atom,
- Sp is an atom or a group of atoms forming a bond between Q and A,
the associative group being chosen from an imidazolidinyl, triazolyl, ureyl, bis-ureyl, ureido-pyrimidyl group.

2. Modified polymer according to Claim 1, **characterized in that** the polymer is a diene elastomer.

3. Modified polymer according to Claim 1 or 2, **characterized in that** the diene elastomer is essentially saturated, preferably selected from ethylene-propylene-diene monomer (EPDM) copolymers and butyl rubbers.

4. Modified polymer according to Claim 1 or 2, **characterized in that** the diene elastomer is essentially unsaturated, preferably selected from natural rubber, synthetic polyisoprenes, polybutadienes, butadiene copolymers, isoprene copolymers and mixtures of these elastomers.

5. Modified polymer according to any one of Claims 1 to 4, **characterized in that** the group A corresponds to one of the following formulae (II) to (VI): 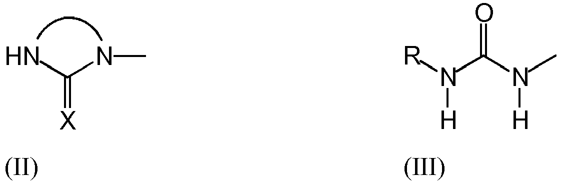 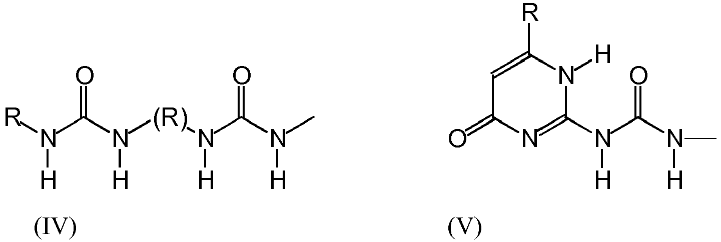 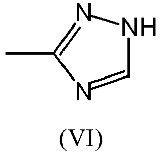 where:
- R denotes a hydrocarbon group which may optionally contain heteroatoms,
- X denotes an oxygen or sulphur atom or a group -NH, preferably an oxygen atom.

6. Modified polymer according to any one of Claims 1 to 5, **characterized in that** the group Q comprises a nitrile oxide, nitrone or nitrile imine functional group.

7. Modified polymer according to any one of Claims 1 to 6, **characterized in that** the group Q is a group of the following formula (VII), (VIII) or (IX) 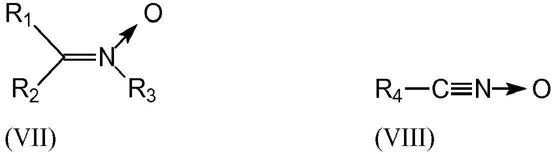 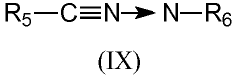 in which R1 to R6 are independently selected from a spacer group Sp, a hydrogen atom, a linear or branched C1-C20 alkyl group, a linear or branched C3-C20 cycloalkyl group, a linear or branched C6-C20 aryl group and a group of formula (X) 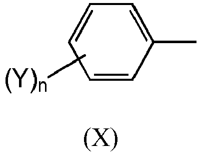 in which n represents 1, 2, 3, 4 or 5 and each Y independently represents a spacer group Sp, an alkyl group or a halide.

8. Modified polymer according to any one of Claims 1 to 7, **characterized in that** the "spacer" group is a linear or branched C1-C24, preferably C1-C10, alkyl chain comprising one or more heteroatoms selected from nitrogen, sulphur, silicon or oxygen atoms.

9. Modified polymer according to any one of Claims 1 to 8, **characterized in that** the group Q is a group of formula (XIa) or (XIb): 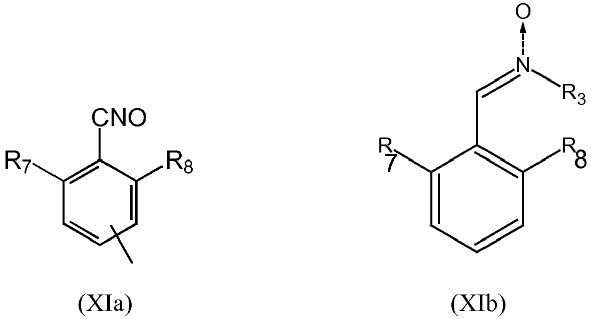 in which R7 and R8 independently represent a C1-C5 alkyl group or a halide and preferably R7 and R8 independently represent a methyl group or a chlorine atom, R3 is as defined in Claim 8 and the group A is a group of formula (XII)

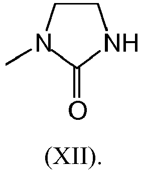

10. Modified polymer according to any one of Claims 1 to 9, **characterized in that** it is chosen from the compounds of the following formulae (XIII) to (XXI): 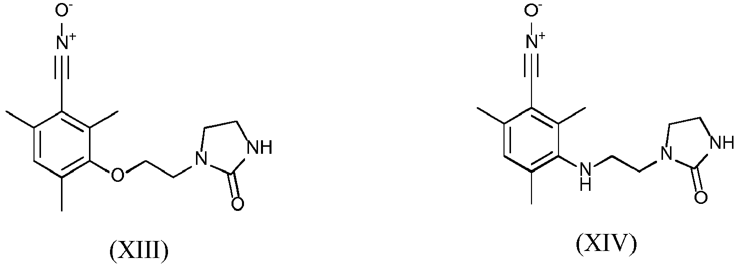 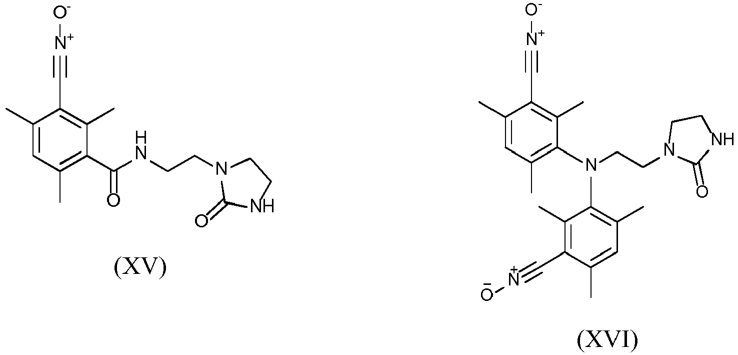 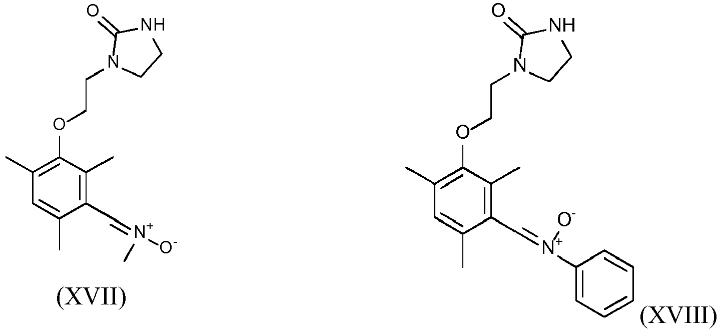 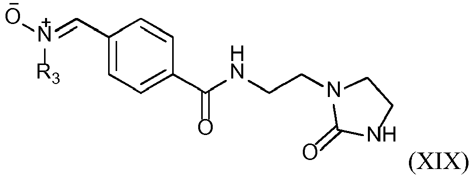 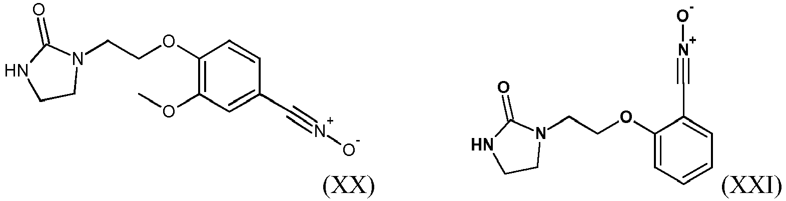 R3 being as defined in Claim 7

11. Modified polymer according to any one of Claims 1 to 8, **characterized in that** it is chosen from the compounds of the following formulae (XXII) and (XXIII): 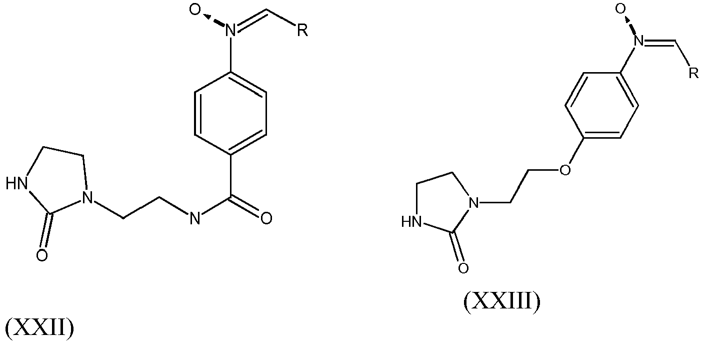 in which R is independently selected from a spacer group Sp, a hydrogen atom, a linear or branched C1-C20 alkyl group, a linear or branched C3-C20 cycloalkyl group, a linear or branched C6-C20 aryl group, and a group of formula (X) 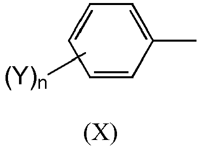 in which n represents 1, 2, 3, 4 or 5 and each Y independently represents a spacer group Sp, an alkyl group or a halide.

12. Process for the preparation of a modified polymer comprising a step for grafting onto a polymer comprising at least one unsaturation of a compound comprising at least one group Q, and at least one group A bonded to each other by at least, and preferably, one "spacer" group Sp as defined in Claims 1 to 11, by [3+2] cycloaddition of the group Q onto the said unsaturation.

13. Process for the preparation of a modified polymer according to Claim 12, **characterized in that** the polymer is a diene elastomer.
